# Europäisches Patentamt
# European Patent Office
# Office européen des brevets

(11) Numéro de publication: **0 366 532 B1**

## (12) FASCICULE DE BREVET EUROPEEN

(45) Date de publication de fascicule du brevet: **21.09.94**

(51) Int. Cl.5: **A61K 37/02**, C12P 21/02, C12N 15/26

(21) Numéro de dépôt: **89402910.7**

(22) Date de dépôt: **23.10.89**

Le dossier contient des informations techniques présentées postérieurement au dépôt de la demande et ne figurant pas dans le présent fascicule.

(54) **Médicaments contenant une interleukine-2 glycosylée.**

(30) Priorité: **21.10.88 FR 8813865**
**18.04.89 FR 8905150**

(43) Date de publication de la demande:
**02.05.90 Bulletin 90/18**

(45) Mention de la délivrance du brevet:
**21.09.94 Bulletin 94/38**

(84) Etats contractants désignés:
**AT BE CH DE ES FR GB GR IT LI LU NL SE**

(56) Documents cités:
EP-A- 0 158 487    EP-A- 0 172 619
EP-A- 0 251 001    EP-A- 0 307 285
WO-A-88/00967    US-A- 4 675 383

**TEBS LETTERS, vol. 226, no. 1, décembre 1987, pages 47-52, Amsterdam, NL; P.FERRARA et al.: "Characterization of recombinant glycosylated human interleukin2 produced by a recombinant plasmid transformed CHO cell line"**

(73) Titulaire: **ELF SANOFI**
**32-34, rue Marbeuf**
**F-75008 Paris (FR)**

(72) Inventeur: **Roskam, Willem**
**Majouret**
**décédé (FR)**
Inventeur: **Basuyaux, Bertrand**
**17, rue Carle Hébert**
**F-92400 Courbevoie (FR)**
Inventeur: **Ferrara, Pascual**
**St. Assiscle - Avignonet**
**F-31290 Villefranche de Lauragais (FR)**
Inventeur: **Laporte, Martine**
**58, rue Romain Rolland**
**F-31520 Ramonville-saint-Agne (FR)**
Inventeur: **Maureaud, Thierry**
**61, les Jardins de Nambours**
**F-31650 Auzielle (FR)**
Inventeur: **Vita, Natalio**
**52, avenue Jules Julien**
**F-31400 Toulouse (FR)**
Inventeur: **Bayol, Alain**
**5, rue de la Sardane**
**F-31170 Tournefeuille (FR)**

Rank Xerox (UK) Business Services
(3.10/3.09/3.3.3)

R.K. SCOPES: "Protein purification, principles and practice", édition 2, 1987,pages 218-220, Springer-Verlag, New York, US

PHARMACIA FPLC; 1986, pharmacia, Uppsala, SE

THE JOURNAL OF IMMUNOLOGY, vol. 141, no. 10, 15 novembre 1988, pages 3671-3679, The American Association of Immunologists, US; R.L. HORNUNG et al.

CHEMICAL ABSTRACTS, vol. 110, no. 7, 1989, page 28, résumé no. 50841k, Columbus, 0hio, US; M. HOSOKAWA et al

Inventeur: **Perry, Geneviève**
**8, Impasse Bourtholle**
**F-31100 Toulouse (FR)**

(74) Mandataire: **Gillard, Marie-Louise et al**
**Cabinet Beau de Loménie**
**158, rue de l'Université**
**F-75340 Paris Cédex 07 (FR)**

## Description

La présente invention concerne une nouvelle interleukine-2 glycosylée, un procédé pour l'isoler à partir du surnageant de culture de cellules CHO recombinées qui sécrètent cette protéine, ainsi qu'un nouveau médicament contenant de l'interleukine-2 glycosylée.

L'interleukine-2 (ci-après partois abrégée IL-2) est une lymphokine sécrétée notamment par les lymphocytes T des mammifères en réponse à une activation par un antigène ou un composé mitogène. Elle joue un rôle essentiel en agissant sur la prolifération et la différentiation de différents types de cellules impliquées dans les réponses immunitaires (Robb R.J. (1984) Immunol. Today, 5, 203-209), ainsi que sur d'autres cellules.

L'interleukine-2 glycosylée d'origine humaine a été plus particulièrement étudiée. On sait qu'elle est produite sous la forme d'une protéine de 133 acides aminés portant, fixée sur le résidu thréonine en position 3, une structure oligosaccharide (Conradt, H.S. et al. (1986) Eur. J. Biochem., 153, 255-261). Les lymphocytes T la synthétisent d'abord sous la forme d'un précurseur de 153 acides aminés et la sécrètent après élimination par coupure, au niveau du réticulum endoplasmique, du peptide-signal de 20 acides aminés, puis après glycosylation dans l'appareil de Golgi, sous la forme d'une protéine de 133 acides aminés -dite protéine mature glycosylée.

Devant les quantités insuffisantes d'interleukine-2 obtenu, soit à partir de lymphocytes périphériques, soit à partir d'une lignée cellulaire lymphoblastoïde telle que la lignée Jurkatt, les techniques de recombinaison génétique sont apparues prometteuses : elles permettent en effet de faire exprimer le gène d'une protéine dans un hôte qui lui est étranger, qu'il s'agisse de cellules eucaryotes ou de cellules procaryotes. Ainsi, le gène de l'interleukine-2 a pu être cloné et exprimé avec succès dans la bactérie Escherichia coli et dans diverses cellules eucaryotes, en particulier les cellules de singe COS et les cellules d'ovaire de hamster chinois CHO.

Le document EP-A-172619 suggère d'isoler, à des fins thérapeutiques, de l'interleukine-2 produite à partir de cellules animales recombinées appelée, dans ce document, "interleukine-2 glycosylée". Selon ce document (voir exemple 3), on prépare de l'"interleukine-2 glycosylée" par immunoprécipitation du surnageant de culture des cellules CHO. Mais d'une part l'interleukine-2 glycosylée n'est pas isolée (elle est obtenue en mélange avec le sérum anti-IL-2 et le tampon détergent NET) et d'autre part cette "interleukine-2 glycosylée" est en fait un mélange d'interleukine-2 glycosylée et d'interleukine-2 non glycosylée, l'immunoprécipitation ne permettant pas de séparer les différentes formes de l'interleukine-2 qui réagissent toutes avec le sérum anti-IL-2.

Le document EP-A-251001 est relatif à la stabilisation d'"interleukine-2 glycosylée humaine" produite à partir de lymphocytes humains stimulés à la phytohémaglutinine. Celle-ci est un mélange de différentes formes d'interleukine-2.

Le document EP-A-158487 décrit la stabilisation d'interleukine-2 d'origine E. coli, donc non glycosylée.

Récemment, la demanderesse a mis au point un procédé de préparation d'interleukine-2 à partir de cellules CHO recombinées permettant un haut niveau d'expression de cette protéine, obtenue principalement sous la forme d'interleukine-2 glycosylée (voir la demande EP-A-307285 et P. Ferrara et al. (1987), Febs letters, 226, 1, 47-52). Elle a donc entrepris des recherches pour isoler de grandes quantités d'interleukine-2 glycosylée.

La séparation et la purification à partir d'un surnageant de culture d'une protéine à usage pharmaceutique ne sont pas des problèmes faciles à résoudre. Il est en effet nécessaire d'éliminer les substances contaminantes liées au système de production, notamment les protéines contaminantes de la souche productrice, les acides nucléiques, les endotoxines, les virus potentiellement présents et les formes dégradées de la protéine isolée. Il faut d'autre part veiller à ne pas dénaturer cette dernière, de façon à conserver son activité et ne pas provoquer des réactions immunitaires parasites chez le patient. De plus, pour une production à l'échelle industrielle, il est souhaitable que le procédé d'isolement de la protéine ne fasse pas appel à des techniques difficiles à mettre en oeuvre telles que l'immuno-affinité et garantisse un bon rendement en protéine isolée par rapport à la protéine présente dans le milieu de culture.

Des procédés de purification de l'interleukine-2 naturelle et recombinante ont été décrits. K. Katok (Katok K (1985) Biochem and Biophys commun, 127, 1, 182-190) par exemple divulgue une méthode de purification de l'interleukine-2 produite à partir de culture de lymphocytes périphériques activés qui comprend la succession des étapes suivantes : chromatographie d'échange cationique, chromatographie d'échange anionique, chromatographie d'exclusion et chromatographie HPLC de phase inverse. M.P. Weir pour sa part (Weir M.P. (1987) J. of Chromatography, 396, p. 209-215) décrit un procédé d'extraction et de purification d'interleukine-2 recombinante dérivée d'E. coli qui comporte les étapes suivantes : lyse des cellules par sonication, extraction au butanol, dissolution des agrégats d'interleukine-2 avec du chlorure de guanidine 8 M en présence de dithiothréitol, chromatographie d'exclusion, renaturation de la protéine par dilution du chlorure de guanidine en présence de sulfate de cuivre, chromatographie d'échange cationique et chromatographie HPLC de phase inverse.

Ces méthodes font intervenir une étape de chromatographie HPLC de phase inverse, laquelle permet d'obtenir un facteur de purification élevé grâce au caractère hydrophobe de la molécule mais nécessite l'emploi de pH acides et de solvants organiques, conditions peu douces susceptibles de dénaturer l'interleukine-2. Elles ne permettent pas de purifier l'interleukine-2 glycosylée présente dans le surnageant de culture des cellules CHO de façon à satisfaire les critères énoncés ci-dessus.

La demanderesse a donc été surprise de constater qu'il était possible d'isoler du surnageant de cellules CHO recombinées une nouvelle interleukine-2 glycosylée présentant des propriétés particulièrement intéressantes au plan pharmaceutique à l'aide d'un procédé simple et utilisable à grande échelle comportant les étapes suivantes : séparation d'une fraction riche en interleukine-2 du surnageant, chromatographie d'échange cationique, chromatographie d'interaction hydrophobe et chromatographie d'exclusion. Cette interleukine-2 glycosylée est constituée d'un mélange d'interleukine-2 glycosylée disialilée et d'interleukine-2 glycosylée monosialilée.

La présente invention a pour objet un médicament qui contient comme principe actif une interleukine-2 glycosylée purifiée constituée d'un mélange d'interleukine-2 glycosylée disialilée et d'interleukine-2 glycosylée monosialilée, seule ou en association avec d'autres principes actifs, dans un excipient pharmaceutiquement acceptable. Avantageusement l'excipient pharmaceutiquement acceptable contient de la gélatine hydrolysée et de l'alanine.

Selon une variante de l'invention, l'interleukine-2 glycosylée disialilée est majoritaire dans l'interleukine-2 purifiée.

L'interleukine-2 glycosylée purifiée selon l'invention est isolée à partir du surnageant de culture de cellules CHO recombinées à l'aide d'un procédé comprenant les étapes suivantes :

a) séparation d'une fraction riche en interleukine-2 du surnageant de culture ;

b) chromatographie d'échange cationique, les fractions $N_1$ et $N_2$ obtenues étant regroupées et soumise aux étapes de :

c) chromatographie d'interaction hydrophobe,

c) chromatographie d'exclusion.

De préférence les cellules CHO recombinées sont des cellules dérivées de la souche DXB 11 par transformation à l'aide d'un vecteur d'expression pour un précurseur de l'interleukine-2 et de la DHFR.

L'interleukine-2 glycosylée purifiée selon l'invention a une activité CTLL-2 supérieure à $15 \times 10^6$ U/mg, proche de l'activité CTLL-2 de l'interleukine-2 naturelle (d'environ $20 \times 10^6$ U/mg). Après lyophilisation de l'interleukine-2 glycosylée solubilisée dans une solution aqueuse à pH 6,5 et reconstitution immédiate du soluté, la solution reconstituée est limpide à un pH physiologique et contient au moins 80 % de l'activité CTLL-2 initiale.

On préfère que les cellules CHO recombinées soient des cellules CHO dhfr- dérivées de la souche DXB 11 par transformation à l'aide d'un vecteur d'expression pour un précurseur de l'interleukine-2 et la DHFR. Un vecteur d'expression préféré dans ce cas est un plasmide ayant les caractéristiques du plasmide pSV 726. Les cellules CHO recombinées ont de préférence été cultivées dans un milieu synthétique pauvre en protéines. Ce dernier peut sans inconvénient contenir de la polyvinylpyrrolidone, composé qui permet d'accroître la productivité spécifique en interleukine-2 des cellules CHO recombinées.

Les cellules CHO recombinées sont cultivées sur les milieux appropriés bien connus de l'homme de l'art et de préférence sur des milieux synthétiques pauvres en protéines, par exemple des milieux contenant environ 4 mg/ml de protéines totales. De préférence ces milieux contiennent de la polyvinylpyrrolidone.

La fraction riche en interleukine-2 est avantageusement séparée du surnageant de culture par double filtration entre une membrane 1 laissant passer cette protéine et une membrane 2 la retenant. La membrane 1 est, par exemple, une membrane de microfiltration ou d'ultrafiltration. La membrane 2 est une membrane d'ultrafiltration. On préfère que la membrane 1 ait un seuil d'arrêt supérieur à 30 kDa, en particulier compris entre 50 et 150 kDa et la membrane 2 ait un seuil d'arrêt inférieur à 10 kDa, de préférence compris entre 5 et 10 kDa. Des matières appréciées pour les membranes 1 et 2 sont l'acétate de cellulose et polysulfone.

On effectue avantageusement l'étape d'échange cationique dans un intervalle de pH compris entre 4,5 et 6,5, en particulier entre 5,2 et 5,7, sur un support chromatographie constitué d'un gel rigide ou semi-rigide à base de polymères hydrophiles, tels qu'un agarose éventuellement réticulé, un polyacrylamide ou un polymère vinylique hydrophile, ou de silice enrobée avec un polymère hydrophile, support sur lequel sont greffés des groupements lui conférant des propriétés d'échangeur de cations fort. Des groupements de ce type appréciés sont les sulfopropyles (SP).

4

L'étape de chromatographie d'échange cationique est de préférence précédée d'une étape de chromatographie d'échange anionique.

Il est intéressant alors d'effectuer l'étape d'échange anionique dans un intervalle de pH compris entre 5,5 et 8,5, en particulier entre 6,5 et 8,2, sur un support chromatographique constitué d'un gel rigide ou semi-rigide à base de polymères hydrophiles, tels que un agarose réticulé, un polyacrylamide ou un polymère vinylique hydrophile, ou de silice enrobée avec un polymère hydrophile, support sur lequel sont greffés des groupements lui conférant des propriétés d'échangeur d'anions faible. Des groupements de ce type appréciés sont les diéthylaminoéthyles (DEAE).

Il est avantageux d'effectuer l'étape d'interaction hydrophobe dans un intervalle de pH compris entre 4,5 et 8,0, en particulier entre 6,0 et 8,0, sur un support chromatographique permettant la désorption de l'IL-2 glycosylée sans addition d'agents chaotropiques ou de solvants organiques ou de solvants aqueux ayant un pH inférieur à 4,5 ou supérieur à 8,0. Des exemples de tels supports sont les gels à base de polymères hydrophiles, tels que un agarose réticulé, ou un polymère vinylique hydrophile, ou de silice enrobée avec un polymère hydrophile, supports sur lesquels sont greffés des groupements butyles, phényles ou propyles.

On effectue de préférence l'étape de chromatographie d'exclusion dans un intervalle de pH compris entre 5,0 et 8,0, en particulier entre 6,0 et 7,0 sur un support ayant une gamme de fractionnement compris entre 1 et 250 kDa. Ce dernier est avantageusement à base de dextrane réticulé avec de l'acrylamide ou à base d'agarose.

L'interleukine-2 glycosylée isolée à l'aide du procédé ci-dessus présente par rapport aux autres interleukines-2 recombinantes purifiées, en particulier celles dérivées d'E. Coli, des propriétés très favorables à son emploi comme principe actif d'un médicament : activité antitumorale supérieure, excellentes propriétés immunostimulantes, toxicité très inférieure, moindre immunogénicité, solubilité dans un solvant aqueux à un pH physiologique sans ajout d'agent solubilisant toxique ni modification chimique de la molécule, excellente stabilité après lyophilisation dans des formulations pharmaceutiquement acceptables.

L'interleukine-2 glycosylée purifiée selon l'invention permet d'obtenir une solution limpide à un pH physiologique, après lyophilisation d'une solution aqueuse la contenant de pH 6,5 et reconstitution immédiate du soluté.

Elle conserve son activité CTLL-2 initiale après lyophilisation dans une solution aqueuse de pH 6,5, additionnée de gélatine hydrolysée ou d'albumine sérique humaine, conservation du lyophilisat à une température de 4° C pendant un an et reconstitution du soluté.

Elle conserve également son activité CTLL-2 initiale après lyophilisation dans une solution aqueuse de pH 6,5, additionnée de gélatine hydrolysée et d'alanine, conservation du lyophilisat à une température de 25° C ou 37° C pendant 3 mois et reconstitution du soluté.

L'interleukine-2 glycosylée selon l'invention pourra être utilisée seule ou en association avec d'autres principes actifs tels que d'autres cytokines, par exemple l'interleukine-1, l'interleukine-4, l'α-interféron, le γ-interféron ou le TNF, des agents antitumoraux, par exemple l'acétate de flavone, le cyclophosphamide, des agents antimitotiques, des immunomodulateurs, par exemple la cyclosporine ou le BCQ, des anticorps ou des hormones thymiques.

Dans toutes les applications, connues ou à découvrir, de l'IL-2, ainsi que dans les associations de l'IL-2 avec d'autres principes actifs, l'IL-2 glycosylée selon l'invention constituera un succédané avantageux de l'IL-2 dérivée d'E. Coli.

Parmi ces applications, on peut citer :
- le traitement de diverses affections telles que le cancer, les immunodéficiences induites ou acquises, les anomalies immununologiques (en particulier les maladies auto-immunes et inflammatoires) et les maladies infectieuses (notamment les infections virales microbiennes et parasitaires) ;
- le traitement prophylactique des affections ci-dessus et en particulier des maladies infectieuses ;
- la vaccination (l'IL-2 étant utilisé comme adjuvant).

L'application en thérapeutique de l'IL-2 glycosylée ainsi que des associations de l'IL-2 glycosylée avec d'autres principes actifs peut être obtenue par son injection intraveineuse bolus ou en perfusion, souscutanée, intramusculaire, intrapéritonéale ou locale par exemple dans des tumeurs, dans le ganglion ou dans d'autre organes immunocompétents mais aussi par son action in vitro sur des cellules prélevées sur le malade ou sur des donneurs.

L'invention sera mieux comprise à l'aide des exemples ci-après.

EXEMPLE 1 : CONSTRUCTION DE VECTEURS D'EXPRESSION POUR UN PRECURSEUR DE L'INTERLEUKINE-2 ET LA DHFR : LES PLASMIDES pSV 720 et pSV 726.

La construction des plasmides comprend notamment l'isolement de fragments d'ADN à l'aide d'enzymes de restriction à partir de vecteurs préexistants, la synthèse par voie chimique d'oligonucléotides, l'assemblage - éventuellement après modification de leurs extrémités - de ces divers fragments en utilisant une enzyme telle que l'ADN-ligase du bactériophage T4, la sélection après clonage - après transformation bactérienne dans Escherichia coli - des plasmides puis leur purification.

Elle fait appel à des techniques bien connues de l'homme de l'art.

Ces techniques sont notamment décrites dans l'ouvrage intitulé Molecular Cloning : a Laboratory Manual de Maniatis, T. et al. publié en 1982 par les Editions Cold Spring Harbor Press à New York (E.U.A.).

L'ensemble des enzymes de restriction nécessaires à la construction des vecteurs ci-après présentés est commercialisé notamment par la Société New England Biolabs (E.U.A.).

L'ADN-ligase du bactériophage T4 est disponible auprès de la Société New England Nuclear (E.U.A.).

a) le plasmide pSV 720

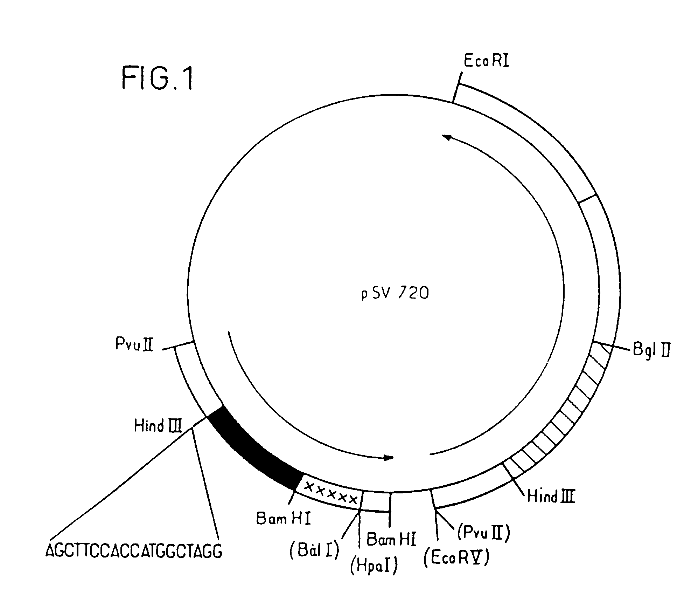

Celui-ci, représenté sur la figure 1, résulte de l'assemblage des 7 fragments d'ADN ci-après par mise en oeuvre des méthodes indiquées précédemment :

- un fragment PvuII-HindIII de 342 paires de bases (ci-après pb) issu du génome SV 40 (Fiers, W. (1978) Nature, 273, 113-120) et contenant le promoteur précoce de ce virus,
- un fragment HindIII-BamHI de 504 pb contenant une séquence d'ADN codant pour le précurseur naturel de l'IL-2 dont la séquence de nucléotides AGCTTCCACAATGTACAGG située à l'extrémité 5′ du brin codant est remplacée par la séquence synthétique AGCTTCCACCATGGCTAGG, de manière à disposer, au niveau des nucléotides encadrant le codon ATG, d'une séquence conforme à la séquence consensus CCACCATGG décrite par Kozak, M. ((1984) Nuc. Ac. Res., 12, 857-872),
- un fragment BamHI-Ball de 305 pb issu du gène de l'alphaglobine de souris (Nishioka, Y. et Leder, P. (1979) Cell, 18, 875-882) et qui contient l'intron distal de ce gène,
- un fragment HpaI-BamHI de 133 pb issu du génome du virus SV 40 et contenant le signal précoce de polyadénylation de ce virus,
- un fragment BamHI-EcoRV de 185 pb issu du plasmide pBR 322 (Bolivar, F. (1977) Gene, 2, 95-113),
- un fragment PvuII-EcoRI de 2677 pb issu du plasmide pSV2-dhfr (Subramani, S. et al (1981) Molecular and Cellular Biology, 1, 854-864) déposé à la collection ATCC sous la référence 37146,
- un fragment EcoRI-PvuII de 2295 pb issu du plasmide pBR 322.

Le plasmide pSV 720 contient :

- une unité d'expression pour le précurseur naturel de l'IL-2. Cette unité a pour promoteur le promoteur précoce du virus SV 40 ; elle comporte, en aval de la séquence codant pour le précurseur de l'IL-2, une séquence qui contient le deuxième intron du gène de l'alphaglobine de souris puis le signal précoce de polyadénylation du virus SV 40.
- une unité d'expression pour la dhfr. Cette unité a pour promoteur le promoteur précoce du virus SV 40 ; elle comporte, en aval de la séquence codant pour la DHFR, la séquence comprise entre les sites MboI en positions 4693 et 4083 - selon la notation de Fiers, W. - sur le génome du virus SV 40 et contenant un intron pour l'antigène t du virus SV 40 puis le signal précoce de polyadénylation du virus SV 40. Cette unité est contenue dans le fragment PvuII-EcoRI issu du plasmide pSV2-dhfr.

b) Le plasmide pSV 726

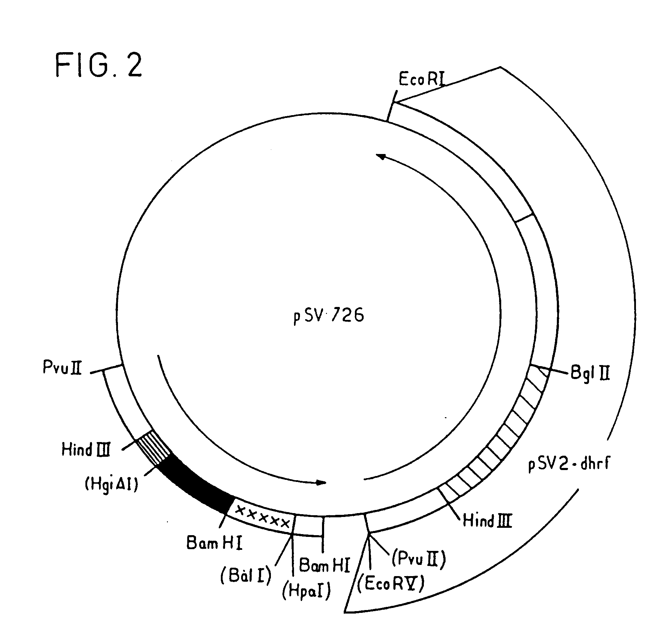

Ce dernier, représenté sur la figure 2, est issu du plasmide pSV 720 de la façon suivante :

Le segment d'ADN compris entre les sites de restriction HindIII et HgiAI situé dans la partie amont du segment HindIII-BamHI du plasmide pSV 720 et contenant la séquence correspondant au peptide-signal modifié (il comporte un résidu alanine en position 2 au lieu du résidu tyrosine en raison de l'adoption d'une séquence conforme à la séquence consensus de Kozak) du précurseur naturel de l'IL-2 et au premier acide aminé de l'IL-2 mature, est ensuite remplacé par un oligonucléotide double brin synthétique, codant à partir de son neuvième nucléotide pour le peptide-signal du précurseur naturel de l'hormone de croissance humaine hGH, ci-après noté peptide-signal de l'hGH, et pour le premier acide aminé de l'IL-2 mature.

Le plasmide obtenu est le plasmide pSV 726, dont le segment HindIII-BamHI porte la séquence codant pour un précurseur de l'IL-2 (le précurseur ps-hGH-IL-2) comportant le peptide-signal de l'hGH.

Le plasmide pSV 726 contient :

- une unité d'expression pour le précurseur (ps-hGH)-Il-2. Cette unité a pour promoteur le promoteur précoce du virus SV 40 ; elle comporte, en aval de la séquence codant pour le précurseur de (ps-hGH)-Il-2, une séquence qui contient le deuxième intron du gène de l'alphaglobine de souris puis le signal précoce de polyadénylation du virus SV 40.

- une unité d'expression pour la dhfr. Cette unité a pour promoteur le promoteur précoce du virus SV 40 ; elle comporte, en aval de la séquence codant pour la dhfr, la séquence comprise entre les sites Mbol en positions 4693 et 4083 - selon la notation de Fiers, W. - sur le génome du virus SV 40 et contenant un intron pour l'antigène t du virus SV 40 puis le signal précoce de polyadénylation du virus SV 40. Cette unité est contenue dans le fragment PvuII-EcoRI issu du plasmide pSV2-dhfr.

La légende suivante a été adoptée pour les figures 1 et 2.

séquence d'ADN issue du plasmide pBR 322

séquence d'ADN issue du génome du virus SV 40

séquence d'ADN issue du gène codant pour l'alphaglobine de souris

séquence d'ADN constituant la séquence codant pour le précurseur naturel de l'interleukine-2 humaine ou son variant dont un résidu alanine a été substitué au résidu tyrosine en position 2

HindIII          BamHI

séquence d'ADN constituant la séquence codant pour le précurseur (ps-hGH)-IL-2

HindIII          BamHI

séquence d'ADN codant pour la dhfr.

EXEMPLE 2 : PREPARATION DE LIGNEES CELLULAIRES HAUTEMENT PRODUCTRICES EN INTERLEUKINE-2 : LES LIGNEES 58-12 et 109-27

Des cellules CHO dhfr⁻ de la souche DXB11 (clone de $CHOK_1$ DHFR⁻ décrit par URLAUB et al dans PNAS USA (1980) 77, 7, p.4216 - 4220) ont été transfectées avec l'un ou l'autre des plasmides pSV 726 et pSV 720.

Le mode opératoire décrit par Graham, F. et Van der Eb, A. ((1973) Virology, 54, 536-539) a été suivi.

Les cellules sont tout d'abord propagées dans du milieu alpha-MEM (Gibco) additionné à raison de 10 % (v/v) de sérum foetal de veau, de 20 $\mu$g/ml de gentamycine, de 60 $\mu$g/ml de tylocine et de 300 $\mu$g/ml de L-glutamine (milieu appelé ci-après milieu non sélectif).

Après une phase de lavage, les cellules ensemencées la veille sont recouvertes de milieu non sélectif et l'on ajoute 10 $\mu$g de l'un des plasmides en présence de phosphate de calcium mais sans ADN de sperme de saumon. Des cellules ainsi préparées sont incubées 7 heures à 37°C.

Les cellules sont ensuite cultivées dans du milieu alpha-MEM additionné, à raison de 5 % (v/v), de sérum foetal de veau pendant 3 jours à 37°C. A l'issue de cette incubation, elles sont réparties à raison de $5.10^5$ cellules par boîte de Petri dans du milieu essentiel minimum commercialisé par la Société Gibco sous la référence 041-1095 ; ce milieu est ici utilisé additionné de sérum foetal de veau dialysé Gibco (10 % v/v), de gentamycine (20 $\mu$g/ml), de tylocine (50 $\mu$g/ml), de L-glutamine (300 $\mu$g/ml) et de L-proline (150 $\mu$g/ml). Ce milieu ainsi complété est dénommé ci-après milieu sélectif.

Les cellules ainsi préparées sont incubées à 37°C pendant 2 semaines en renouvelant le milieu sélectif tous les 3 jours. Les colonies observées à l'issue de cette incubation sont en principe issues de cellules ayant effectivement incorporé un plasmide. Ces colonies sont prélevées et remises en culture séparément dans du milieu sélectif et testées par une mesure de l'activité de type IL-2 pour s'assurer de leur aptitude à produire de l'IL-2.

Cette activité de type IL-2 est l'activité biologique du milieu de culture selon le test de stimulation de la prolifération de la lignée de lymphocytes T de souris IL-2 dépendante CTLL-2 (Baker, P. et al., J. Exp. Med., 1979, 149-173). Cette activité sera appelée par la suite activité CTLL-2 et exprimée en Unité (abrégée U par la suite) par rapport au standard défini dans le cadre du B R M P (Biological Res. Modifiers Program (1984), Lymphokin Res. 4, 193-227).

Les colonies les plus productrices ont été repiquées successivement dans quatre préparations de milieu sélectif présentant l'une par rapport à la précédente une concentration accrue (0,02, 0,05, 0,1 puis 0,2 uM) en méthotrexate (amethopterin, Sigma) de la manière décrite par Alt. F. et al ((1978 Journal of Biological Chemistry, 253, 1357-1570).

C'est ainsi qu'ont pu être retenues la lignée CHO 58.12 transformée par le plasmide pSV 720 et la lignée 109.27 transformée par le plasmide pSV 726, hautement productrices en interleukine-2.

EXEMPLE 3 : <u>MISE EN CULTURE DES LIGNEES HAUTEMENT PRODUCTRICES EN INTERLEUKINE-2 :</u> <u>LIGNEES DE CELLULES CHO 58-12 et 109-27</u>

**I - PRODUCTION D'IL-2 AVEC LA LIGNEE CHO 58-12 DANS UN MILIEU DE CULTURE MOYENNEMENT RICHE EN PROTEINES**

Après obtention d'un nombre suffisant de cellules par propagation en boîtes rollers (flacons tournants), un fermenteur de production est inoculé à raison de 300 000 cellules/ml environ. Les conditions de fermentation sont les suivantes :

**a)** culture en perfusion avec un taux de perfusion de 1 volume par jour environ ;

**b)** le surnageant est recueilli en continu après passage à travers un spin-filter (filtre tournant solidaire de l'arbre d'agitation) ;

**c)** culture sur microbilles de dextran réticulé recouvertes d'une pellicule de collagène dénaturé (Cytodex III R, commercialisé par la Société PHARMACIA) ;

**d)** pH de culture régulé à 7,3 environ ;

**e)** pression en oxygène dissous régulée à 30 % (le 100 % ayant été défini comme la saturation du milieu en oxygène après barbotage d'air) ;

**f)** température de la culture régulée à 37°C environ ;

**g)** le milieu de culture de base appelé milieu 1, est principalement à base du mélange suivant (50 : 50) : milieu essentiel minimum d'Eagle MEM (FLOW) et milieu nutritif F12 de Ham (GIBCO).

La composition du milieu 1 est donnée ci-après :

| ACIDES AMINES | PM | mg/l | mM |
|---|---|---|---|
| L- Alanine | 89,09 | 13,35 | 0,15 |
| L- Arginine. HCl | 210,7 | 168,5 | 0,8 |
| L- Asparagine. $H_2O$ | 150 | 22,5 | 0,15 |
| L- Cystine. 2HCl | 313,2 | 15,65 | 0,05 |
| L- Cysteine. HCl. $H_2O$ | 175,6 | 17,55 | 0,10 |
| L- acide glutamique | 147 | 22,05 | 0,15 |
| L- Glutamine | 146 | 292 | 2 |
| L- Glycine | 75,07 | 11,25 | 0,15 |
| L- Histidine. HCl. $H_2O$ | 209,7 | 31,48 | 0,15 |
| L- Isoleucine | 131,2 | 27,97 | 0,21 |
| L- Leucine | 131,2 | 32,55 | 0,25 |
| L- Lysine. HCl | 182,7 | 54,5 | 0,30 |
| L- Methionine | 149,2 | 9,74 | 0,065 |
| L- Phenylalanine | 165,2 | 18,5 | 0,11 |
| L- Proline | 115 | 28,75 | 0,25 |
| L- Serine | 105 | 15,75 | 0,15 |
| L- Threonine | 119,1 | 30 | 0,25 |
| L- Tyrosine | 181,2 | 29,89 | 0,165 |
| L- Tryptophane | 204,2 | 6 | 0,03 |
| L- Valine | 117,2 | 28,85 | 0,246 |
| L- acide aspartique | 133 | 19,95 | 0,15 |

| SELS INORGANIQUES | PM | mg/l | mM |
|---|---|---|---|
| $CaCl_2$ | 110,99 | 86,5 | 0,78 |
| KCl | 74,56 | 311,8 | 4,18 |
| $KH_2PO_4$ | 136,09 | 30 | 0,22 |
| $FeSO_4 . 7H_2O$ | 278,02 | 1,8 | $6,5\ 10^{-3}$ |
| $MgSO_4$ | 120,37 | 48,84 | 0,4 |
| NaCl | 58,44 | 7800 | 133 |
| $NaHCO_3$ | 84,01 | 350 | 4,1 |
| $MgCl_2$ | 95,22 | 28,5 | 0,3 |
| $Na_2HPO_4$ | 141,96 | 94,94 | 0,67 |

| OLIGOELEMENTS | PM | mg/l | mM |
|---|---|---|---|
| $CuSO_4 . 5H_2O$ | 249,68 | $1,5\ 10^{-3}$ | $6\ 10^{-6}$ |
| $MnSO_4 . H_2O$ | 169,02 | $0,845\ 10^{-3}$ | $5\ 10^{-6}$ |
| $Na_2SeO_3 . 5H_2O$ | 263,01 | $3,42\ 10^{-2}$ | $1,4\ 10^{-4}$ |
| $NiCl_2 . 6H_2O$ | 237,70 | $1,19\ 10^{-6}$ | $5\ 10^{-9}$ |
| $(NH_4)_6\ Mo_7O_{24} . 4H_2O$ | 1235,89 | $1,235\ 10^{-3}$ | $1\ 10^{-6}$ |
| $NH_4\ VO_3$ | 116,99 | $5,845\ 10^{-4}$ | $5\ 10^{-6}$ |
| $SiO_2\ Na_2O . 5H_2O$ | 212,14 | 0,1067 | $5\ 10^{-4}$ |
| $SnCl_2 . 2H_2O$ | 225,63 | $1,12\ 10^{-6}$ | $5\ 10^{-9}$ |
| $Zn\ SO_4 . 7H_2O$ | 287,54 | 2,13 | $7\ 10^{-3}$ |

EP 0 366 532 B1

| VITAMINES | PM | mg/l | mM |
|---|---|---|---|
| Biotine | 244,3 | $3,65 \ 10^{-3}$ | $0,015 \ 10^{-3}$ |
| Chlorure de choline | 139,6 | 7,48 | 0,05 |
| Acide folique | 441,4 | 2,45 | 0,003 |
| Nicotinamide | 122,13 | 0,513 | $4,6 \ 10^{-3}$ |
| Pyridoxal. HCl | 203,63 | 0,5 | $2,5 \ 10^{-3}$ |
| Pyridoxine. HCl | 205,6 | 0,031 | $3 \ 10^{-4}$ |
| Riboflavine | 376,4 | 0,069 | $2 \ 10^{-4}$ |
| Thiamine. HCl | 337,3 | 0,67 | $2 \ 10^{-3}$ |
| Cyanocobalamine | 1355,4 | 0,68 | $5 \ 10^{-4}$ |
| Pantothenate de calcium D | 238,3 | 0,74 | $3 \ 10^{-3}$ |

| AUTRES CONSTITUANTS | PM | mg/l | mM |
|---|---|---|---|
| D. Glucose | 18 | 1800 | 10 |
| Rouge de phénol (sel sodique) | | 11 | |
| Pyruvate de Sodium | 110 | 110 | 1 |
| Hypoxanthine (sel sodique) | 136,1 | 2,38 | $1,75 \ 10^{-2}$ |
| Acide lipoique | 206,3 | 0,105 | $5 \ 10^{-4}$ |
| Acide Linoléique | 278,44 | 0,042 | $1,5 \ 10^{-4}$ |
| Putrescine 2HCl | 161,1 | 0,0805 | $5 \ 10^{-4}$ |
| i-Inositol | 180,2 | 10 | $5,5 \ 10^{-2}$ |
| Thymidine | 242,2 | 0,365 | $1,5 \ 10^{-3}$ |
| MOPS | 209,3 | 6279 | 30 |

La fermentation se décompose en deux parties appelées respectivement phase de croissance et phase de production. Pendant la phase de croissance, le milieu est complété avec du sérum de veau foetal (5 %). Pendant la phase de production, ce milieu est complété avec une fraction protéique, appelée F IV-1, isolée à partir de sérum bovin selon le procédé de fractionnement de Cohn. Le milieu de culture est un milieu moyennement riche en protéines (400 mg de protéines totales/l). La production est maintenue pendant au moins 30 jours. La récolte est refroidie en ligne à une température de +6°C. La concentration en protéines totales est de 500 mg/l. L'activité CTLL-2 dans le surnageant de culture est de 40 000 U/ml, soit une concentration d'IL-2 d'environ 2 mg/l (si on admet une activité spécifique de l'IL-2 de $2 \times 10^7$ U/mg et une pureté protéique en IL-2 de 0,4 %. Ce surnageant de culture sera dénommé ci-après surnageant de culture A.

## II - PRODUCTION D'IL-2 AVEC LA LIGNEE CHO 109-27 DANS UN MILIEU PAUVRE EN PROTEINES

Après obtention d'un nombre suffisant de cellules par propagation en boites Rollers (flacons tournants) un fermenteur de production est inoculé à partir de celui-ci à raison de 300 000 cellules/ml environ. Les conditions de fermentation sont les suivantes :

**a)** culture en perfusion avec un taux de perfusion de 1 volume par jour environ ;

**b)** le surnageant est recueilli en continu après passage à travers un spin-filter ;

**c)** culture sur microbilles de dextran réticulé recouvertes d'une pellicule de collagène dénaturé (Cytodex III R, commercialisé par la Société PHARMACIA) ;

**d)** pH de culture régulé à 7,3 environ ;

**e)** pression en oxygène dissous régulée à 100 % ;

**f)** température de la culture régulée à 37°C environ ;

**g)** le milieu de culture de base est le milieu 1 défini ci-dessus.

La fermentation se décompose en deux parties appelées respectivement phase de croissance et phase de production. Pendant la phase de croissance, le milieu est complété avec du sérum de veau foetal (2,5 %). Pendant la phase de production, le sérum est remplacé par 3 mg/l d'insuline et 1 mg/l de la lactoferrine comme seules protéines du milieu.

Pendant cette phase, on ajoute en plus 0,5 % de polyvinylpyrrolidone d'un poids moléculaire moyen de 40.000 au milieu. On a en effet constaté que ce polymère permettait d'accroître la productivité spécifique

10

en IL-2 des cellules CHO recombinées (quantité d'IL-2 secrétée par unités de temps et de biomasse). Le milieu de culture est donc un milieu synthétique pauvre en protéines (4 mg/l de protéines totales). La production est maintenue pendant au moins 30 jours. La récolte est refroidie en ligne à une température de +6°C. La concentration en protéines totales du surnageant de culture est de 100 mg/l. L'activité CTLL-2 est de 120 000U/ml, soit une concentration d'environ 6 mg/l et une pureté protéique de 6 %. Ce surnageant de culture sera dénommé ci-après surnageant de culture B.

EXEMPLE 4 : CARACTERISATION PARTIELLE DE L'IL-2 SECRETEE PAR LES LIGNES DE CELLULES CHO 58-12 ET 109-27

**1.** PURIFICATION DE L'IL-2

L'IL-2 est purifiée à partir d'un litre de surnageant de culture.

On procède tout d'abord à une concentration et à une première purification en soumettant le surnageant à une chromatographie d'échange d'ions sur une colonne d'agarose Sepharose[R] (S-Fast Flow - Pharmacia Fine Chemical - Suède) préalablement équilibrée avec de l'acétate d'ammonium 0,05 M à pH 4,5. L'élution est réalisée à l'aide d'acétate d'ammonium 0,05 M (pH 5,5) additionné de NaCl de molarité 0,05 M puis 0,5 M.

Les fractions éluées, qui s'avèrent biologiquement actives d'après une mesure de leur activité CTLL-2, sont rassemblées et leur pool est soumis à une chromatographie liquide à haute performance sur une colonne de phase inverse. Le support choisi est un gel de silice greffée en $C_3$. La colonne a pour dimensions : 1,0 x 25,0 cm.

L'élution est réalisée, avec un gradient linéaire d'acétonitrile variant de 5 à 100 % (v/v) dans une solution dans l'eau à 0,1 % (v/v) d'acide trifluoroacétique, en 80 min, avec un débit de 4 ml/min.

Les fractions éluées, biologiquement actives, sont rassemblées et leur pool est soumis à une chromatographie de même type que celle ci-dessus réalisée, mais dans des conditions notamment d'élution identiques, sur un gel de silice greffée en $C_{18}$ dans une colonne de dimensions : 2,1 x 10,0 cm.

Le pool des fractions éluées recueillies lors de cette chromatographie, biologiquement actives, et présentant, d'après les résultats d'une électrophorèse sur gel de polyacrylamide en présence de dodécylsulfate de sodium (Laemli, (1970) Nature, 277, 680-685), une pureté en IL-2 supérieure à 95 % constitue le matériel sur lequel est effectuée la caractérisation de l'IL-2.

**2.** CARACTERISATION DE L'IL-2 PAR DETERMINATION DE LA SEQUENCE AMINO-TERMINALE

Les échantillons à traiter sont portés à la surface d'un filtre de bromure d'hexadiméthrine (ou polybrene). Le filtre est introduit dans un séquenceur de protéines (modèle 470 A commercialisé par la Société Applied Biosystems (E.U.A.)) équipé d'un chromatographe (modèle 430 A Applied Biosystems) qui analyse en continu les dérivés phénylthiohydantoïques formés, après chaque cycle de dégradation.

Les résultats de cette détermination sont en accord avec la séquence déjà publiée pour le produit naturel (Robb, R. et al. (1984) Proc. Natl. Acad. Sci. U.S.A., 81, 6486-6490), sauf en ce qui concerne la position 3 où aucun acide amine n'a été détecté. Cette non-détection s'explique par la présence d'un oligosaccharide en position 3.

Cette séquence s'écrit pour ses dix premiers acides aminés :

```
              1                                          10
      Ala-Pro-Thr-Ser-Ser-Ser-Thr-Lys-Lys-Thr
```

L'alanine est le seul résidu détecté en position N terminale. Cela apporte la confirmation que le précurseur (ps-hGH)-IL-2 a été correctement coupé lors de la sécrétion.

EXEMPLE 5 - ISOLEMENT DE L'INTERLEUKINE-2 GLYCOSYLEE A L'AIDE DU PROCEDE DE L'INVENTION A PARTIR DU SURNAGEANT DE CULTURE DE LA LIGNEE CHO 109-27

Dans cet exemple et dans l'exemple 7, l'eau utilisée est de l'eau déminéralisée purifiée sur un appareil de type Milli-Q et ultrafiltrée.

## 1 - Séparation du milieu de culture d'une fraction riche en interleukine-2

On prélève 130 litres du surnageant du milieu B décrit dans l'exemple 3 et on les préfiltre sur un filtre de seuil d'arrêt 8 $\mu$m de façon à éliminer les grosses particules susceptibles de colmater les membranes d'ultrafiltration.

On fractionne et on concentre ensuite l'interleukine-2, dont le poids moléculaire est compris entre 15 kDa et 17 kDa, par double ultrafiltration tangentielle entre une première membrane de seuil d'arrêt 100 kDa et une deuxième membrane de seuil d'arrêt 10 kDa, en opérant de la façon décrite ci-après. Les première et deuxième membranes sont des cartouches spirales en acétate de cellulose, les membranes YM 100 et YM 10 commercialisées par la Société AMICON, montées en cascade de façon que le filtrat issu de la première membrane alimente le rétentat de la deuxième membrane.

On envoie le préfiltrat sur la première membrane et on concentre d'abord le rétentat de la première membrane ainsi que celui de la deuxième membrane jusqu'à obtenir un volume de rétentat de la première membrane d'environ 15 litres. Puis on diafiltre (lave à volume constant) les rétentats avec 80 litres d'eau ultrapurifiée, de façon à épuiser en IL-2 le rétentat de la première membrane et faire baisser la force ionique de celui de la deuxième membrane. Ensuite, on concentre ce dernier jusqu'à 1,5 litre et on récupère l'interleukine-2 après rinçage de la membrane à l'eau. On filtre le concentrat obtenu à travers un filtre de seuil d'arrêt 0,2 $\mu$m de façon à éliminer certains précipités formés au cours de l'ultrafiltration. On obtient ainsi 2,4 litres de solution aqueuse concentrée en IL-2.

## 2 - Isolement de l'interleukine-2 glycosylée

### a) Chromatographie d'échange anionique

Cette étape permet d'éliminer par fixation sur support chromatographique une partie des contaminants tels que les acides nucléiques, les endotoxines et les protéines de CHO et les autres protéines présentes dans le surnageant de culture. L'IL-2 n'est pas retenue sur la colonne dans les conditions d'élution choisies. Le support chromatographique utilisé est un gel à base d'agarose réticulé d'échange d'anions faible, le DEAE Sepharose fast flow commercialisé par la Société PHARMACIA, placé dans une colonne de verre de diamètre 140 mm sur une hauteur de 210 mm. On ajoute à la solution concentrée en IL-2 obtenue précédemment des cristaux d'acétate d'ammonium de façon à ajuster sa conductivité à 5 mS/cm et on l'injecte dans le support chromatographique préalablement équilibré avec une solution tampon d'acétate d'ammonium de pH 7. Puis on lave la colonne avec cette dernière solution. On réunit l'effluent de fixation et la solution de lavage qui contiennent l'IL-2. On obtient ainsi 7,08 litres de solution aqueuse riche en IL-2.

### b) Chromatographie d'échange cationique

Cette étape permet d'éliminer une partie des contaminants tels que la polyvinylpyrrolidone, les protéines de CHO et les autres protéines présentes dans le surnageant de culture, non fixés sur le support chromatographique anionique et de séparer les différentes formes de l'IL-2.

Le support chromatographique utilisé est un gel à base de résine polyvinylique hydrophile, le SP Fractogel 650 (S) commercialisé par la Société MERCK, placé dans une colonne de diamètre 50 mm sur une hauteur de 230 mm. On ajuste à 5,5 le pH de la solution obtenue à l'étape précédente par ajout à cette dernière d'acide acétique et on l'injecte sur la colonne. On élue ensuite l'IL-2 fixée sur la colonne par une solution de force ionique croissante obtenue en mélangeant dans des proportions différentes une solution aqueuse d'acétate d'ammonium 50 mM de pH 5,5 avec une solution aqueuse de chlorure de sodium 2 M. On mesure la densité optique de la solution à 280 nm. On sépare ainsi trois fractions contenant cette protéine :
- la fraction 1 contenant l'IL-2 glycosylée disialilée (forme $N_2$)[1]
- la fraction 2 contenant l'IL-2 glycosylée monosialilée (forme $N_1$)[1]
- la fraction 3 contenant l'IL-2 glycosylée non sialilée (forme $N_o$)[1] et l'IL-2 non glycosylée (forme M)[1]

En réunissant les fractions 1 et 2, on obtient 0,45 litre de solution aqueuse de IL-2 glycosylée.

(1) cf. H. Conradt (1985), Eur. J. Biochem. 153, 255-261 pour une description des différentes formes de l'IL-2.

c) Chromatographie d'interaction hydrophobe

Le support chromatographique utilisé est un gel d'interaction hydrophobe à base de résine polyvinylique hydrophile, le Butyl Fractogel 650 (M) commercialisé par la Société MERCK, placé dans une colonne de diamètre 70 mm sur une hauteur de 135 mm.

On ajoute à la solution d'IL-2 glycosylée obtenue précédemment du sulfate d'ammonium jusqu'à une molarité de 1,2 M et de l'ammoniaque pour ajuster son pH à 6,5 et on l'injecte dans le support chromatographique, préalablement équilibré avec une solution aqueuse de pH 6,5 et de molarité 50 mM en phosphate d'ammonium et 1,2 M en sulfate d'ammonium. L'IL-2 glycosylée est retenue dans ces conditions sur le support chromatographique. On élimine les produits non retenus par lavage avec successivement la solution précédente et une solution aqueuse de pH 6,5 de molarité 50 mM en phosphate d'ammonium et 0,8 M en sulfate d'ammonium. On élue ensuite l'IL-2 avec une solution aqueuse de pH 6,5 50 mM de phosphate d'ammonium et 0,1 M de sulfate d'ammonium. On obtient ainsi 2,17 litres de solution aqueuse d'IL-2 glycosylée.

d) Chromatographie d'exclusion

Le support de chromatographie d'exclusion est un gel à base de dextrane réticulé dont le domaine de fractionnement est compris entre 250 et 5 kDa, le Sephacryl S 200 HR commercialisé par la Société PHARMACIA, placé dans une colonne de diamètre 100 mm sur une hauteur de 900 mm.

Après concentration de la solution obtenue à l'issue de l'étape c) sur une membrane spirale en acétate de cellulose de seuil d'arrêt 10 kDa, on l'injecte dans la colonne préalablement équilibrée avec une solution aqueuse 50 mM de phosphate de sodium de pH 6,5. On élue ensuite l'IL-2 glycosylée avec cette dernière solution. Celle-là est détectée en sortie de colonne par mesure de la densité optique à 280 nm, les différentes fractions collectées étant réunies d'après leur pureté analysée par HPLC de phase inverse.

On obtient ainsi 0,60 litre d'une solution d'IL-2 glycosylée dans du phosphate de sodium.

3 - Vérification de la pureté du produit obtenu

**\* Electrophorèse sur gel de polyacrylamide en présence de SDS**

Une électrophorèse sur gel de polyacrylamide PAGE-SDS (Sodium Dodécyl Sulfate) en présence de réducteur (dithiothréitol) a été réalisée selon la méthode de LAEMMLI (LAEMMLI, U.K. Anal. Biochem, (1977), 78, p. 459) et suivie d'une coloration à l'argent selon la méthode de MERRIL (MERRIL, C.R., Proc. Nat. Acad. Sci. USA, 1979, vol. 76, p. 4335).

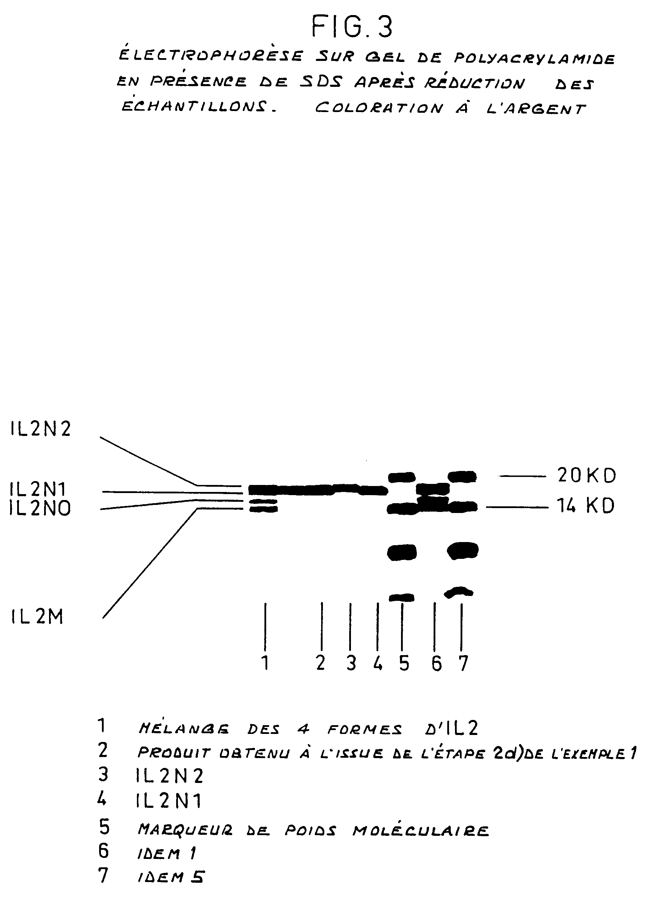

L'électrophorégramme obtenu, reproduit sur la figure 3, permet d'affirmer que la pureté en IL-2 glycosylée est supérieure à 99,5 %.

**\* Analyse par HPLC sur colonne de phase inverse**

Le support chromatographique est un gel de silice greffée, le Nucleosil $C_4$ 300 A de granulométrie 5 um commercialisé par la Société Française de Chromato-colonne. L'élution est réalisée avec un gradient de 6 minutes de (70 % A + 30 % B) à (25 % A + 75 % B), A désignant une solution aqueuse contenant 0,1 % d'acide trifluoroacétique et B une solution d'acétonitrile contenant 0,1 % d'acide trifluoroacétique.

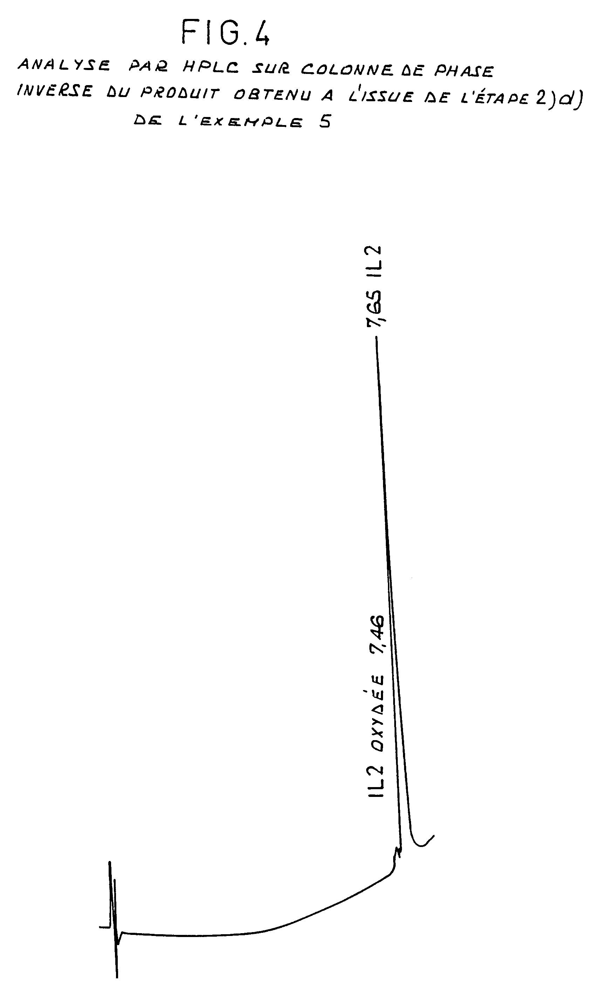

Le chromatogramme obtenu, reproduit sur la figure 4, montre la présence d'une seule impureté représentant environ 5,5 % de la quantité du produit. Celle-là a été partiellement caractérisée : il s'agit d'IL-2 glycosylée oxydée qui présente également l'activité CTLL-2.

**\* Analyse par HPLC sur colonne d'échange de cations**

Le support chromatographique est un gel de silice enrobé d'un acide poly-aspartique, le "polycat A 5 $\mu$m 300 A", commercialisé par la Société CLUZEAU. L'élution est réalisée avec un gradient de 3,5 mm de (85 % A' + 15 % B') à (75 % A' + 25 % B') et de 6,5 mm de (75 % A' + 25 % B') à (50 % A' + 50 % B'), A' désignant une solution de pH 5,0 de molarité 25 mM en $KH_2PO_4$ et B' une solution de pH 5,0 de molarité 25 mM en $KH_2PO_4$ et 1 M en chlorure de sodium.

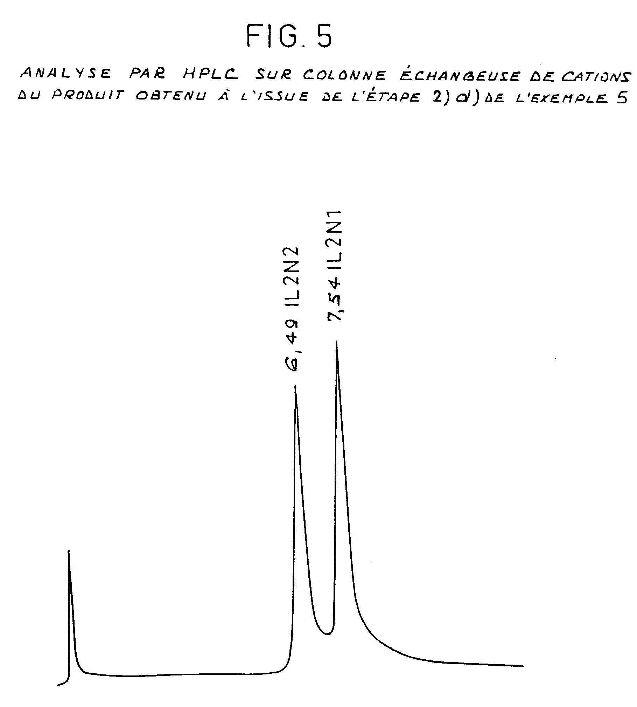

Le chromatogramme obtenu, reproduit sur la figure 5, permet de quantifier les deux formes de l'IL-2 glycosylée présentes : 48 % d'IL-2($N_2$) et 52 % d'IL-2($N_1$). Dans d'autres expériences, la forme $N_2$ est majoritaire.

4 - <u>Bilan</u>

La quantité d'IL-2 présente dans le produit retenu a été mesurée à l'issue de chacune des étapes d'isolement par dosage de l'activité CTLL-2 de ce dernier. Les différentes formes de l'IL-2 ont la même activité CTLL-2.

Les résultats sont rassemblés dans le tableau 1 ci-après.

TABLEAU 1

| Produit retenu à l'issue de l'étape | Volume (l) | Activité CTLL-2 totale ($10^{10}$ U) | Activité CTLL-2 volumique (U/ml) | Rendement |
|---|---|---|---|---|
| Production (surnageant) | 127 | 1,521 | $1,2 \times 10^5$ | |
| 1- | 2,4 | 1,290 | $4,8 \times 10^6$ | 85 % |
| 2- a) | 7,08 | 1,212 | $1,2 \times 10^6$ | 94 % |
| 2- b) | 0,45 | 0,982 | $2,2 \times 10^7$ | 81 % |
| 2- c) | 2,17 | 0,874 | $3,6 \times 10^6$ | 89 % |
| 2- d) | 0,60 | 0,751 | $1,2 \times 10^7$ | 86 % |
| Rendement global = 49,4 % | | | | |

Ce tableau montre que l'activité CTLL-2 par unité de volume est 100 fois plus concentrée à l'issue de l'étape 2- d) que dans le surnageant de culture et que le rendement global en IL-2 glycosylée est d'environ 50 % par rapport à l'IL-2 totale présente dans le surnageant.

EXEMPLE 6 : CARACTERISATION DE L'INTERLEUKINE-2 GLYCOSYLEE ISOLEE A L'AIDE DU PROCEDE DE L'INVENTION A PARTIR DU SURNAGEANT DE CULTURE DE LA LIGNEE CELLULAIRE CHO 109.27

- détermination de l'activité spécifique CTLL-2 = $(18 \pm 3) \times 10^6$ U/mg

- détermination de la séquence aminoterminale

Les échantillons de produit sont portés à la surface d'un filtre de bromure d'hexadiméthrine (ou de polybrene). Le filtre est introduit dans un séquenceur de protéines (modèle 470 A, commercialisé par la Société Applied Biosystems (E.U.A.)) équipé d'un chromatographe (modèle 430 A - Applied Biosystems) qui analyse en continu les dérivés phénylthiohydantoïques formés.

Les résultats de cette détermination sont en accord avec la séquence déjà publiée pour le produit naturel (Robb, R. et al. (1984) Proc. Natl. Acad. Sci. U.S.A., 81, 6486-6490), sauf en ce qui concerne la position 3 où aucun acide aminé n'a été détecté. Cette non-détection s'explique par la présence d'un oligosaccharide en position 3.

Cette séquence s'écrit pour ses six premiers acides aminés :

```
       1                                              10
Ala-Pro-Thr-Ser-Ser-Ser-Thr-Lys-Lys-Thr
```

- détermination de la structure primaire

Le produit, réduit et carboxyméthylé, a été digéré pendant la nuit à température ambiante sous l'action de la trypsine ajoutée dans une proportion de 1/30 (poids/poids) et les peptides obtenus ont été séparés par chromatographie HPLC de phase inverse au moyen d'un gradient d'acétonitrile (en présence de 0,1 % d'acide trifluoroacétique) selon la méthode publiée dans le Journal of Immunological Methods (1985), 81, 15-30.

Chaque peptide tryptique purifié a fait l'objet d'une analyse d'acides aminés et pour certains d'une dégradation Edman.

Il a ainsi été possible d'analyser la séquence complète d'aminoacides du produit isolé. Celle-ci est en accord avec celle déjà publiée pour le produit naturel (Robb, R. et al. (1984) Proc. Natl. Acad. Sci. U.S.A., 81, 6486-6490).

L'étude des cartes de peptides tryptiques du produit obtenu après réaction avec la vinyl-4-pyridine avant et après réduction du produit avec le dithiothréitol met en évidence un pont disulfure entre la cystéine 58 et la cystéine 105 ainsi qu'un groupe thiol libre (SH) pour la cystéine 125.

- Analyse des oligosaccharides

L'électrophorèse sur gel polyacrylamide en présence de SDS a montré la présence de deux formes du produit de poids moléculaires d'environ 16,5 et 16,0 kDa correspondant aux formes $N_1$ et $N_2$ (cf. figure 3). Le traitement à la neuraminidase de celui-ci (Ferrara P. et al. FEBS (1987), 226, 1, 47-52) a fait disparaître les deux bandes observées et apparaître une bande d'un poids moléculaire de 15,5 kDa correspondant à la forme $N_o$ (cf. figure 3), ce qui indique que les différences entre les deux sont dues à la présence d'acides sialiques dans la molécule. L'analyse du produit par chromatoélectrofocalisation a permis de confirmer ce résultat et de déterminer les points isoélectriques de ces deux constituants : 7,0 pour la forme de poids moléculaire 16,5 kDa et 7,6 pour la forme de poids moléculaire 16 kDa.

- Caractérisation de la structure des sucres

L'analyse des sucres est poursuivie par le traitement de la substance correspondant à la bande de 15,5 kDa (provenant du traitement à la neuramidinase) avec une o-glucanase spécifique (la o-glucanase commercialisée par GENZYME sous la référence 20 O-ASE). Ce traitement enzymatique réduit le poids moléculaire de la substance de 15,5 kDa à 15,0 kDa sans changement de la charge de la substance. Finalement, les peptides tryptiques N terminaux purifiés de la forme $N_1$ et de la forme $N_2$ ont été analysés par FAB MS (Fast Atom Bombardment Mass Spectrometry). Les résultats obtenus ont confirmés la présence de la N-acetylgalactosamine, du galactose et de deux acides neuraminiques sur le peptide tryptique N-terminal de la forme $N_2$ et la présence de la N-acétylgalactosamine, du galactose et d'un acide neuraminique sur le peptide tryptique N-terminal de la forme $N_1$. La structure des sucres pour les formes $N_1$ et $N_2$ de l'IL-2 glycosylée de l'invention est identique à la structure des sucres des formes $N_1$ et $N_2$ décrites par Conradt et al. (1985), Eur. J. of Biochemistry, 153, 255-261.

- Propriétés immunologiques de l'IL-2 glycosylée

Il existe un rapport entre la conformation d'une protéine et ses propriétés antigéniques. L'étude de ces dernières donne donc des indications sur la structure tridimensionnelle de la molécule.

Les propriétés antigéniques de l'IL-2 glycosylée ($N_1$ + $N_2$) et de l'IL-2 naturelle (BIOTEST-RFA) et de l'IL-2 coli préparée comme décrit dans l'exemple 8) ont été étudiées de façon comparative avec deux systèmes RIA (Radio-Immuno-Assay). Dans le premier (RIA A), on a utilisé un anticorps monoclonal anti IL-2 naturelle (anticorps BG5 fourni par le Centre Régional de Transfusion Sanguine de Besançon), dans le deuxième (RIA B) un antisérum de mouton anti IL-2 coli (lot NO Pol-1/00-09 de CELLTECH UK.). Dans les deux cas, le traceur a été l'IL-2 ($N_2$) marquée à l'iode 125.

Le tableau 2 ci-après indique les valeurs de $IC_{50}$ (concentration en IL-2 capable de déplacer 50 % de l'IL-2 ($N_2$) marquée de l'anticorps considéré) obtenues à partir des courbes de déplacement pour chacun de ces systèmes (les valeurs et les écarts types indiqués ont été calculés à partir de trois déterminations indépendantes de ce paramètre).

TABLEAU 2

| $IC_{50}$ avec le système RIA A (pM) | $IC_{50}$ avec le système RIA B (pM) |
|---|---|
| IL-2 glycosylée ($N_1$ ± $N_2$) 57 ± 9 | 124 ± 18 |
| IL-2 coli 135 ± 6 | 204 ± 18 |
| IL-2 naturelle (BIOTEST-RFA) 58 ± 6 | 118 ± 18 |

Il apparaît que les valeurs $IC_{50}$ obtenues pour l'IL-2 glycosylée ($N_1 + N_2$) et de l'IL-2 naturelle sont semblables alors que celles-ci sont différentes de façon significative de celles observées pour l'IL-2 coli, résultat particulièrement net et fiable avec le système RIA A où l'anticorps est monoclonal. Cette observation montre une ressemblance de structure tridimentionnelle entre l'IL-2 glycosylée ($N_1 + N_2$) et l'IL-2 naturelle et une différence de structure tridimensionnelle entre l'IL-2 glycosylée ($N_1 + N_2$) et l'IL-2 coli, lesquelles possèdent donc des déterminants antigéniques différents. Or, un des problèmes rencontrés avec l'IL-2 coli est son immunogénicité (Krigel R.L. et al. (1988) Cancer, Res, 48, 3875-3881 et Allegretta M. et al. (1986) J. of Clin. Immun. 6, 6, 481-490). Ceux-ci sont selon toute vraisemblance dus à des épitopes spécifiques de l'IL-2 coli et absents de l'IL-2 naturelle. Il est très probable, et les résultats ci-dessus le confirment, que l'IL-2 glycosylée, proche par sa structure et son mode de synthèse de l'IL-2 naturelle, n'a pas ces épitopes.

EXEMPLE 7 : ISOLEMENT DE L'INTERLEUKINE-2 GLYCOSYLEE A L'AIDE DU PROCEDE DE L'INVENTION A PARTIR DU SURNAGEANT DE CULTURE DE LA LIGNEE CELLULAIRE 58.12

1 - Séparation du surnageant de culture d'une fraction riche en interleukine-2

On prélève 200 litres de surnageant de culture A décrit dans l'exemple 3. On sépare et on concentre une fraction riche en IL-2 par double ultrafiltration entre une première membrane en polysulfone de seuil d'arrêt 100 kDa et une deuxième membrane en polysulfone de seuil d'arrêt 10 kDa. Cette opération est réalisée de la même façon que dans l'exemple 5 ci-dessus. On obtient ainsi 5,6 litres de solution aqueuse concentrée en IL-2.

2 - Isolement de l'interleukine-2 glycosylée

a) Chromatographie d'échange cationique

Le support chromatographique utilisé est un gel à base d'agarose réticulé d'échange de cations fort , le "S. Sepharose fast flow" commercialisé par la Société PHARMACIA.
On ajuste à 5,5 le pH de la solution obtenue à l'étape précédente par ajout à cette dernière d'acide acétique et on règle sa conductivité à 5 mS par dilution. On injecte alors cette solution sur la colonne, laquelle est ensuite lavée successivement avec différentes solutions aqueuses de pH 5,5 de molarité 50 mM en acétate d'ammonium et de force ionique croissante en chlorure de sodium. L'IL-2 glycosylée est éluée avec une solution 180 mM de chlorure de sodium. On obtient 5,76 litres de solution aqueuse d'IL-2 glycosylée.

b) Chromatographie d'interaction hydrophobe

Le support chromatographique utilisé est un gel d'interaction hydrophobe à base de résine polyvinylique hydrophile, le "Butyl Fractogel 650 (M)" commercialisé par la Société MERCK, placé dans une colonne de 50 mm de diamètre.
On ajoute à la solution d'IL-2 glycosylée obtenue précédemment de l'acétate d'ammonium jusqu'à une molarité de 1,6 M et de l'ammoniaque pour ajuster son pH à 6,5 et on l'injecte dans la colonne, préalablement équilibrée avec le tampon A, solution aqueuse de pH 6,5 de molarité 1,6 M en acétate d'ammonium. L'Il-2 glycosylée est retenue dans ces conditions sur le support chromatographique. On lave la colonne avec le tampon A pour éliminer les produits non retenus. On élue cette dernière avec le tampon B, solution de pH 6,5 de molarité 0,65 M en acétate d'ammonium.
On obtient ainsi 1,36 litre de solution aqueuse d'IL-2 glycosylée.

c) Chromatographie d'exclusion

Le support chromatographique est un gel à base de dextrane réticulé, le "Sephacryl S 200 HR" commercialisé par la Société PHARMACIA.
Après concentration de la solution obtenue à l'issue de l'étape b) sur une membrane en acétate de cellulose, la membrane YM 10 commercialisée par la Société AMICON, de seuil d'arrêt 10 kDa, on l'injecte sur la colonne, préalablement équilibrée avec une solution aqueuse de pH 6,5 de molarité 0,1 M en phosphate de sodium. On élue ensuite l'IL-2 glycosylée avec cette dernière solution.

3 - <u>Mise en évidence de la pureté du produit</u>

Une électrophorèse sur gel de polyacrylamide PAGE-SDS (Sodium Dodecyl Sulfate) en présence de réducteur (dithiothréitol), réalisée selon la méthode de LAEMMLI (LAEMMLI, U.K. Anal. BIOCHEM. 78, 1977) et suivie d'une coloration à l'argent selon la méthode de MERRIL (MERRIL C.R., Proc. Nat. Acad. Sci. USA, vol. 76, p. 4335, 1979), a montré une pureté en IL-2 glycosylée supérieure à 99 %.

<u>EXEMPLE 8</u> - <u>ACTIVITE IN VITRO DE L'INTERLEUKINE-2 GLYCOSYLEE SUR LES LYMPHOCYTES HUMAINS</u> :

Etude comparative de l'interleukine-2 glycosylée et de l'interleukine-2 recombinante dérivée d'<u>E. coli</u> dans la génération des cellules LAK

Les essais décrits dans cet exemple et les exemples suivants ont été réalisés avec l'IL-2 glycosylée recombinante produite par la souche 109.27, isolée comme décrit dans l'exemple 5. Ce produit sera appelé en général IL-2 glycosylée et en cas de risque de confusion IL-2 glycosylée ($N_1$ + $N_2$).

1 - <u>Intérêt en cancérologie de l'étude de la génération des cellules LAK in vitro</u>

L'immunothérapie adoptive représente une nouvelle approche thérapeutique du cancer qui a pour but d'amplifier les réactions de défense immunologique de l'hôte vis-à-vis de la tumeur.

La culture de leucocytes humains du sang périphérique en présence de l'IL-2 confère à ces cellules une puissante capacité cytotoxique contre les cellules tumorales. Les cellules effectrices sont dénommées LAK ("Lymphokine Activated Killers") (LOTZE M.T. et al. (1981) Cancer. Res., 41, 4420-4426). L'action des cellules LAK est dirigée contre tout type de tumeurs y compris les cellules de tumeurs fraîchement prélevées. Elles n'exercent aucune activité dirigée contre les cellules normales. Les cellules LAK sont fonctionnellement définies comme toute cellule qui développe après stimulation par l'IL-2 une cytotoxicité non MHC ("Major Histocomptability Complex") restreinte, dirigée notamment contre des cellules tumorales résistantes à l'action des cellules NK ("Natural Killers") sans sensibilisation antigénique préalable. Cette définition distingue clairement des cellules LAK les cellules NK (spontanément cytotoxiques sans activation par l'IL-2) et les cellules T cytotoxiques dont l'action est MHC restreinte et nécessite une primosensibilisation avec la cible (TALMADGE J.E. et al. (1986) Cancer Treat. Rep., 70, 171-1982).

Des travaux récents suggèrent en réalité que des cellules LAK sont probablement des cellules NK potentialisées par l'IL-2 et que les précurseurs LAK dérivent donc en fait de "Large granular lymphocytes" (ITOH K. et al. (1986) J. Immunol., 136, 3910-3917).

L'interleukine-2 génère non seulement <u>in vitro</u> mais également <u>in vivo</u> des cellules LAK. La capacité d'une IL-2 particulière de générer des cellules LAK in vitro représente donc un indice important de son activité anti-tumorale potentielle.

2 - <u>Méthode expérimentale</u>

a) <u>Les interleukines-2 testées</u>

- l'IL-2 glycosylée ($N_1$ + $N_2$)
- l'IL-2 glycosylée ($N_2$), obtenue en soumettant la fraction 1 isolée au cours de l'étape 2b de l'exemple 5 aux étapes c) et d) de ce dernier ;
- l'IL-2 non glycosylée (M), obtenue en soumettant la fraction 3 isolée au cours de l'étape 2b de l'exemple 5 aux étapes c) et d) de ce dernier ;
- l'IL-2 recombinante dérivée d'<u>E. coli</u>, ci-après parfois abrégée IL-2 coli, préparée comme décrit ci-après.

Le gène codant pour l'IL-2 a été transfecté dans des cellules <u>E. coli</u> et a été exprimé à des taux élevés. Après solubilisation, renaturation et purification par chromatographie HPLC en phase inverse et chromatographie d'exclusion selon la méthode décrite par Liang et al. (Liang S. I et al., (1985) Biochem. J. 229, 429-439), la protéine recombinante a montré une activité spécifique de 5-10 x $10^6$ U/mg.

b) <u>Préparation des lymphocytes</u>

Le milieu de culture utilisé était constitué de RPMI 1640 (Gibco-BRL) additionné de 10 % de sérum humain AB (CTS Purpan, Toulouse, France) inactivé par la chaleur (1 heure à 56°C), 2 mM de pyruvate de sodium, 5 mM d'HEPES, 4 mM de L-glutamine, 100 U/ml de pénicilline, 100 $\mu$g/ml de streptomycine et 0,25 $\mu$g/ml d'amphotéricine B (ce milieu sera désigné par la suite comme milieu complet).

Le sang d'un sujet sain a été prélevé aseptiquement.

Les lymphocytes périphériques ont été séparés des érythrocytes et des granulocytes par centrifugation sur gradient de Ficoll-Hypaque (PHARMACIA) selon la méthode décrite par A. Boyum dans "Methods in enzymology" (G. Di Sabato, J.J. Langone, H. Van Vunakis, ed.), vol. 108, p. 88, Academic Press, Inc., 1984.

Les lymphocytes ont été lavés 3 fois en milieu complet.

Les cellules adhérentes (monocytes et macrophages) ont été éliminées par adhérence sur plastique : les cellules ont été suspendues dans le milieu complet à la concentration de 5 à 10 x $10^6$ par ml et ensemencées dans des flacons de culture à une densité de 1 à 2 x $10^6$ cellules par $cm^2$. Les flacons ont été placés à 37°C en présence de 5 % de $CO_2$ pendant 1 heure au bout de laquelle les lymphocytes non adhérents ont été récupérés par aspiration après agitation douce des flacons de culture.

### c) Culture in vitro des lymphocytes en présence d'IL-2

Les lymphocytes non adhérents isolés au paragraphe précédent ont été lavés une fois et mis en culture à la concentration de $10^6$ cellules par ml dans le milieu complet en présence de différentes concentrations de chacune des deux IL-2 testées (100, 30, 10 et 1 U/ml) à 37°C et en présence de 5 % de $CO_2$ pendant une durée de 48 heures. Les cellules ont ensuite été lavées. Elles sont considérées par la suite comme les cellules effectrices.

### d) Mise en évidence de l'activité cytotoxique

L'activité cytotoxique des cellules effectrices est évaluée après 4 heures de contact avec des cellules cibles de la lignée lymphoïde T humaine C8166-45/C63 décrite par S.Z. Salahuddin et al. (Virology 129, 51-64, (1983) et Science 223, 703-707, (1984)), résistante à une cytotoxicité de type NK. Cette lignée sera appelée par la suite lignée $HT_1$. 6 x $10^6$ cellules cibles sont radiomarquées avec 200 $\mu$Ci de $^{51}Cr$ (chromate de sodium, Amersham) pendant 1 heure à 37°C dans un volume de 0,4 ml de milieu complet sans sérum puis lavées plusieurs fois. Les cellules cibles ($10^4$ dans un volume de 0,1 ml de milieu complet) et les cellules effectrices dans un volume de 0,1 ml de milieu complet sont distribuées dans des plaques de microtitration à fonds ronds (Falcon) à raison de plusieurs rapports cellules effectrices sur cellules cibles (50:1, 10:1, 1:1). Les plaques de microtitration sont centrifugées et après 4 heures d'incubation à 37°C en présence de 5 % de $CO_2$, le surnageant de chaque puits est récupéré et la radioactivité mesurée à l'aide d'un compteur gamma. La cytotoxicité est déterminée par la quantité de $^{51}Cr$ libéré par les cellules cibles mortes. La cytotoxicité non spécifique est déterminée par la quantité de radioactivité spontanément libérée par les cellules cibles en l'absence de cellules effectrices. La cytotoxicité maximum est déterminée par la quantité de radioactivité libérée par les cellules cibles en l'absence de cellules effectrices et en présence d'acide chlorhydrique 1 N. Chaque point expérimental est triplé (sextuplé pour la détermination des cytotoxicités non spécifique et maximum) et le pourcentage de lyse spécifique est calculé comme la moyenne plus ou moins l'écart type selon la formule :

**Pourcentage de lyse spécifique**

$$\frac{\text{cpm des puits expérimentaux} - \text{cpm non spécifique}}{\text{cpm maximum} - \text{cpm non spécifique}} \times 100$$

Le protocole décrit dans les paragraphes b), c) et d) ci-dessus a été suivi pour l'IL-2 glycosylée ($N_2$) et l'IL-2 non glycosylée (M). Un protocole peu différent décrit par H.D. Engers (Engers H.D. et al. (1986) Methods in enzymology, 132, 437-457) a été suivi pour l'IL-2 glycosylée ($N_1$ + $N_2$) et l'IL-2 coli, lesquelles ont été essayées avec des cellules cibles des lignées $HT_1$, Daudi et Jurkatt, toutes résistantes à la lyse NK.

3 - Résultats

Les résultats obtenus sont rassemblés dans les tableaux 3 et 4 ci-après.

TABLEAU 3

| Activité cytolytique contre la lignée $HT_1$ des cellules LAK générées à partir de donneur sain par l'IL-2 non glycosylée (M) et l'IL-2 glycosylée ($N_2$) | | | |
|---|---|---|---|
| Doses d'IL-2 activité CTLL-2 | Rapport cellules effectrices/cellules cibles | Pourcentage de lyse spécifique avec l'IL-2 non glycosylée (M) | Pourcentage de lyse spécifique avec l'IL-2 glycosylée ($N_2$) |
| 1 000 U/ml | 50/1<br>10/1<br>1/1 | 43,6 ± 1,2<br>24,1 ± 0,6<br>6,5 ± 7,9 | 53,9 ± 1,6<br>36,3 ± 3,2<br>33,4 ± 2,4 |
| 100 U/ml | 50/1<br>10/1<br>1/1 | 30,9 ± 2,6<br>13,7 ± 1,8<br>0 | 40 ± 6,1<br>15,4 ± 2,8<br>5,6 ± 4,1 |
| 10 U/ml | 50/1<br>10/1<br>1/1 | 11,3 ± 1,7<br>0<br>0 | 23 ± 3,9<br>7,1 ± 2,2<br>2,9 ± 4,2 |
| 1 U/ml | 50/1<br>10/1<br>1/1 | 0<br>0<br>0 | 7,8 ± 3,9<br>8,5 ± 3,8<br>0,4 ± 0,8 |

## TABLEAU 4

Activités cytolytiques contre les lignées Daudi,

$HT_1$ et Jurkatt des cellules LAK générées par plusieurs

interleukines-2 à partir de lymphocytes de donneurs sains

| Cellules cibles | | Concentration d'IL-2 | |
|---|---|---|---|
| | | 20 U/ml | 50 U/ml |
| Daudi | IL-2 coli | 1 * | 1 * |
| | IL-2 glycosylée $(N_1 + N_2)$ | 3,12 | 1,1 |
| $HT_1$ | IL-2 coli | 1 | 1 |
| | IL-2 glycosylée $(N_1 + N_2)$ | 8 | 1,9 |
| Jurkatt | IL-2 coli | 1 | 1 |
| | IL-2 glycosylée $(N_1 + N_2)$ | 2,7 | 0,95 |

\* Les résultats sont exprimés en index d'activité par rapport à l'IL-2 recombinante coli, défini par :

$$\text{Index d'activité} = \frac{\text{pourcentage de lyse obtenu avec IL-2 coli}}{\text{pourcentage de lyse obtenu avec l'IL-2 glycosylée}}$$

Le tableau 3 montre pour des cellules cibles de la lignée $HT_1$ :
- la supériorité de l'IL-2 glycosylée $(N_2)$ pour des concentrations d'IL-2 inférieures à 1 000 U/ml ;
- cette supériorité augmente lorsque le rapport cellule effectrice/cellule cible diminue ;
- cette supériorité diminue pour de fortes doses d'IL-2 (1 000 U/ml) et/ou pour un rapport cellules effectrices/cellules cibles élevé.

Le tableau 4 montre pour des cellules cibles des lignées Daudi, $HT_1$ et Jurkatt :
- la supériorité de l'IL-2 glycosylée par rapport à l'IL-2 coli à une concentration en IL-2 de 20 U/ml ;
- cette supériorité diminue lorsque la concentration d'IL-2 augmente.

Ces études montrent que :
- l'IL-2 glycosylée génère des cellules LAK actives contre trois lignées tumorales ;
- cette activité LAK est obtenue avec des doses moins importantes avec l'IL-2 glycosylée qu'avec l'IL-2 coli ;
- l'activité des cellules LAK générées par l'IL-2 glycosylée est supérieure à l'activité des cellules LAK générées par l'IL-2 coli dans deux situations :
  * petite dose d'IL-2
  * rapport cellule effectrice/cellule cible défavorable (inférieur à 10/1).

Ces résultats sont particulièrement prometteurs pour l'utilisation clinique de l'IL-2 glycosylée puisque ces études montrent que cette forme d'IL-2 peut être utilisée à des doses plus faibles que l'IL-2 coli pour un effet antitumoral similaire. La toxicité liée à l'administration du produit devrait donc être sensiblement réduite.

EXEMPLE 9 - ACTIVITE IMMUNOMODULATRICE DE L' INTERLEUKINE-2 GLYCOSYLEE : STIMULATION DE LA REPONSE IMMUNITAIRE A MEDIATION HUMORALE CHEZ LA SOURIS

a) But de l'étude

Cette étude a été motivée d'une part parce qu'il a été montré que l'IL-2 coli stimule in vivo une réponse immunoglobuline M (ci-après abrégée IgM) polyclonale chez des souris non immunisées et une réponse IgM spécifique chez des souris immunisées (C.M. Wegand (1986), J. Exp. Med. 163, 1607-1612) et d'autre part que l'IL-2 coli est capable d'induire in vitro sur des cellules humaines la différenciation des lymphocytes B activés (T.A. Waldmann et al. (1984), J. Exp. Med. 160, 1450-1466). Elle a pour but l'appréciation de l'activité in vivo de l'interleukine-2 glycosylée dans la stimulation de la réponse immunitaire à médiation humorale chez la souris BALB/C, par mesure de la réponse splénique primaire à une immunisation avec un antigène thymo-dépendant (l'ovalbumine).

b) Protocole expérimental

α) Animaux - Conditions d'environnement

* Espèce : souris BALB/C
* Acclimatation : les animaux ont été soumis à une période d'acclimatation d'une semaine avant le début de traitement
* Nombre d'animaux : 90 femelles
* Poids des animaux en début d'étude : 20 g environ
* Alimentation : les animaux ont reçu à volonté de l'aliment composé complet A04.c, commercialisé par la Société V.A.R
* Abreuvement : l'eau de ville a été distribuée à volonté par un système d'abreuvement automatique
* Conditions d'environnement :
  - température à 22°C (± 2°C)
  - hygrométrie à 60 % (± 10 %)
  - photopériode 12/24 h
* Habitat : les animaux étaient entretenus dans des cages en acier inoxydable. Ils ont été répartis en groupes de 15 de même lot stabulés en cages de 5 individus.

β) Traitement des animaux

Les animaux ont été immunisés avec de l'ovalbumine de pureté électrophorétique 98 % (commercialisée par la Société SIGMA sous la référence A-5378) au jour 1 de l'étude par voie intrapéritonéale, à raison de 50 μg d'ovalbumine en solution dans 0,5 ml de tampon PBS (Phosphate Buffer Saline) stérile) par souris. L'ovalbumine (poids moléculaire : 40 000, 5 déterminants antigéniques connus) a été choisie comme antigène car c'est une protéine xénogénique qui présente une bonne immunogénicité.

L'IL-2 glycosylée isolée comme décrit à l'exemple 5 a été administrée aux jours 2, 4, 6 et 8 par voie intrapéritonéale dans du sérum normal de souris BALB/C dilué au $80^{ème}$ dans du tampon PBS. La présence de protéines dans ce sérum évite toute perte d'IL-2 par adsorption non spécifique sur les parois des tubes ou des seringues sans influer significativement sur le système immunitaire. Le tableau ci-après précise les doses administrées aux différents lots de 15 souris.

| Lot n° | Dose administrée (U/kg) | Volume administré |
|--------|-------------------------|-------------------|
| 0 | véhicule | 0,5 ml/souris |
| 1 | IL-2/7,5 $10^4$ | 0,5 ml/souris |
| 2 | IL-2/3,75 $10^5$ | 0,5 ml/souris |
| 3 | IL-2/1,88 $10^6$ | 0,5 ml/souris |
| 4 | IL-2/9,38 $10^6$ | 0,5 ml/souris |
| 5 | IL-2/4,69 $10^7$ | 0,5 ml/souris |

Les animaux ont été sacrifiés au jour 9.

γ) Mesures effectuées

Après pesage des cadavres d'animaux, leurs rates ont été prélevées stérilement, pesées et broyées mécaniquement. Le broyat a été filtré sur gaze stérile afin d'éliminer tous les agrégats conjonctivo-adipeux et centrifugé 10 minutes à 400 g. Les splénocytes ont été mis en suspension et lavés trois fois. Après lavage, une série de comptages a été réalisée pour calculer le nombre total des splénocytes par animal et répartir ces derniers à la concentration cellulaire désirée. Les splénocytes ont été mis en culture pendant 96 heures dans des puits de 5 ml de plaques en polystyrène à raison de 2 x $10^6$ splénocytes vivants par ml de milieu de culture, lequel comprend 10 % de sérum de veau foetal (milieu RPMI, 1640 commercialisé par la Société BIOPRO).

Les IgM anti-ovalbumines ont ensuite été dosés par radioimmunométrie de la façon suivante :
* Adsorption sur plaque PVC de l'ovalbumine (100 $\mu$l par puits d'une solution à 50 $\mu$g/ml dans un tampon phosphate 0,1 M pH 7,5), 18 heures à +4°C.
* Après lavage des puits avec du tampon phosphate 0,1 M pH 7,5, saturation des puits pendant 1 heure 30 à +37°C, avec 200 $\mu$l d'une solution de surfactif Tween 20 1 % (v/v) dans le tampon phosphate 0,1 M pH 7,5.
* Après lavage des puits, incubation avec 100 ul par puits de surnageant de culture à doser dilué au 1/2 dans du tampon phosphate 0,1 M pH 7,5 avec 0,1 % (p/v) de gélatine et 0,1 % (v/v) de surfactif Tween 20, 18 heures à +4°C.
* Après lavage des puits, incubation avec 100 ul par puits d'anticorps polyclonal de chèvre anti IgM de souris (distribué par Immunotech sous la référence 115-0575) radiomarqué à l'iode 125 (7,5 ½Ci/½g, 250 000 cpm/100 ul, 3 heures à +37°C).
* Après lavage des puits avec une solution, NaCl 0,9 % (p/v) Tween 20 0,1 % (v/v), la radioactivité fixée a été mesurée à l'aide d'un compteur gamma.

Les différentes mesures rapportées ci-dessous, effectuées pour chacune des souris, ont permis de calculer pour chaque lot de souris donc chaque dose administrée la valeur pondérale moyenne de la rate rapporté au poids corporel, le nombre total moyen de splénocytes par animal et la quantité moyenne d'IgM anti-ovalbumine produite par les splénocytes, cette dernière étant proportionnelle à la radioactivité.

c) Résultats

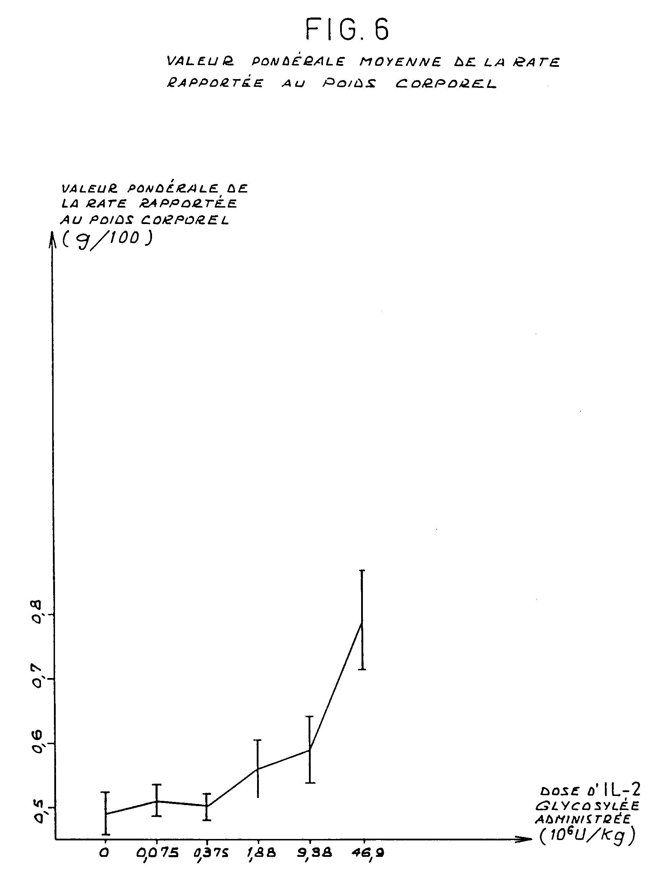

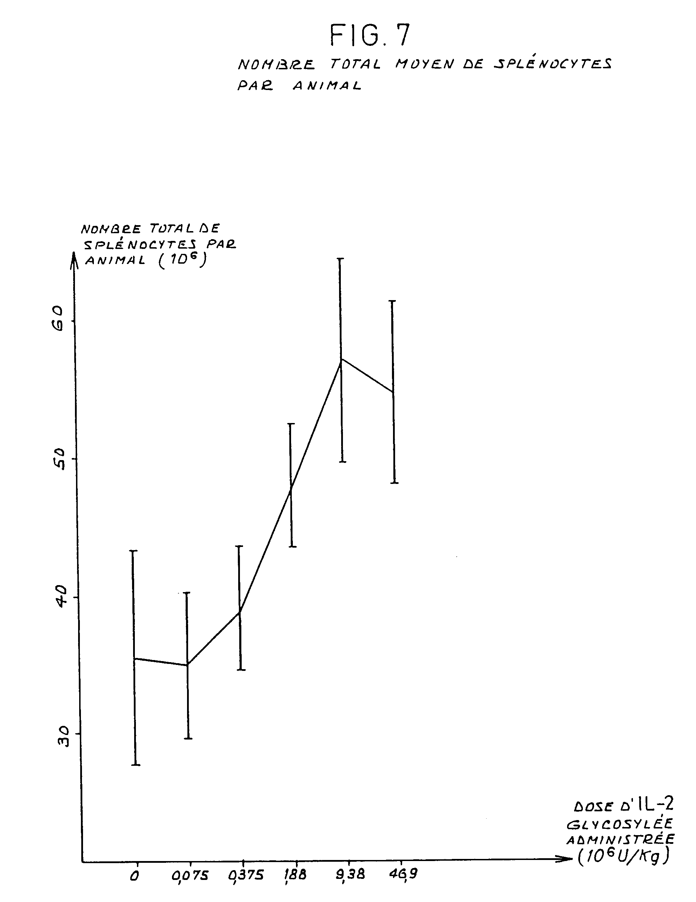

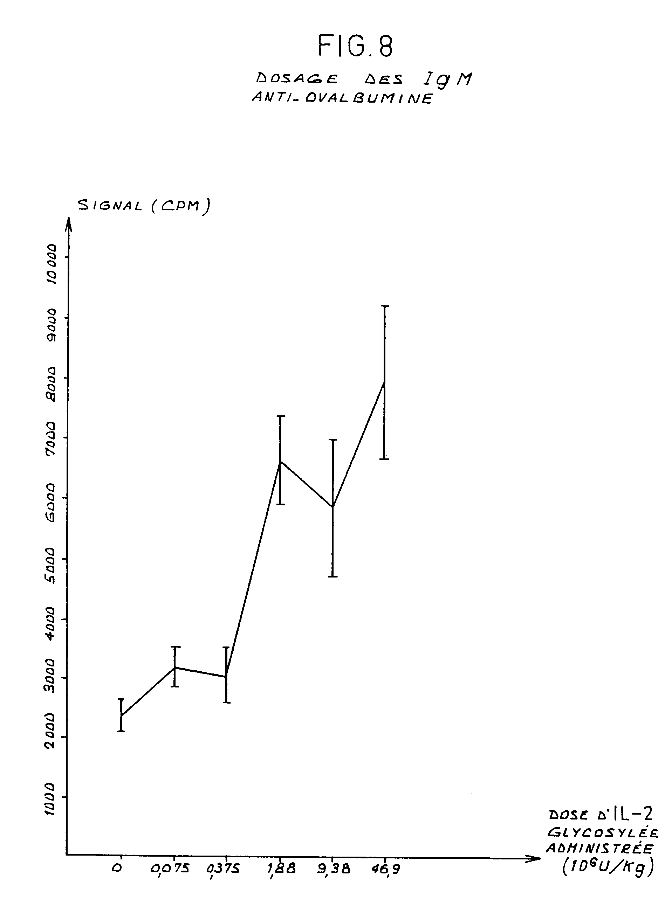

Les résultats obtenus sont représentés sur les figures 6, 7 et 8. On constate au regard de celles-ci :
- à partir de la dose 1,88 x $10^6$ U/kg (soit 37 500 U par souris) l'IL-2 glycosylée accroît de plus de 50 % en valeur relative le poids de la rate et le nombre total de splénocytes par animal. Il a d'ailleurs été montré par des essais non rapportés ici que les lymphocytes T totaux augmentent plus en proportion que les autres (20 %).
- une dose supérieure à 1,88 x $10^6$ U/kg augmente de manière significative la quantité des IgM spécifiques produite par chaque plasmocyte.

L'IL-2 glycosylée a donc une activité immunostimulante importante.

EXEMPLE 10 - ACTIVITE ANTITUMORALE DE L'INTERLEUKINE-2 GLYCOSYLEE : IMMUNOTHERAPIE DU CANCER CHEZ LA SOURIS

a) Méthode expérimentale

L'étude a porté sur des souris DBA/2 réparties en lots de cinq souris. Au jour 0, 0,2 x $10^4$ cellules syngéniques de lymphosarcome SL2 ont été injectées dans les souris par voie intrapéritonéale.

# EP 0 366 532 B1

Dans une première série d'expériences, différentes doses d'interleukine-2 glycosylée isolée comme décrit dans l'exemple 5 (0, 8, 40, 200, 1 000 et 5 000 U/jour) ont été injectées aux souris de 6 lots différents de cinq souris aux jours 3-7 et 10-14. Le nombre de souris survivantes chaque jour pendant une période de 30 jours a été compté. Cette première série d'expériences a permis de montrer une activité antitumorale de l'interleukine-2 glycosylée administrée à la dose de 5 000 U/jour.

Dans une deuxième série d'expériences, on a fixé la dose administrée à 5 000 U pendant les jours 10 à 14 et on a fait varier les doses administrées précédemment ainsi que les jours d'administration de ces dernières.

b) Résultats

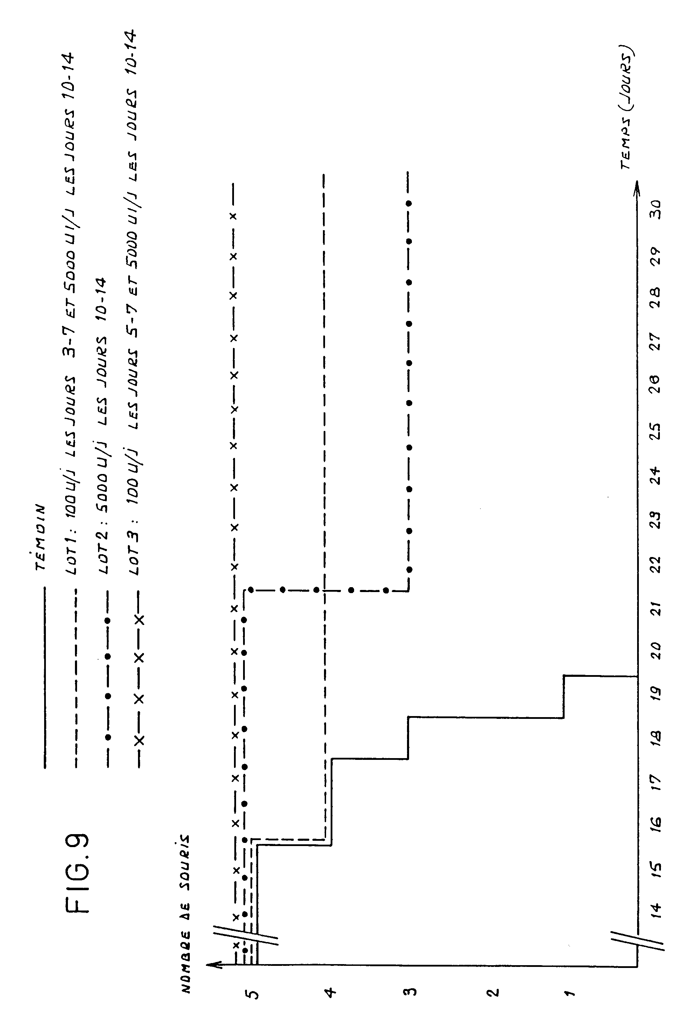

Les résultats de la deuxième série d'expériences sont rassemblés dans le tableau 5 ci-après et illustrés sur la figure 9. Celle-ci représente le nombre de souris survivantes au cours d'une période de 30 jours pour les lots 0, 1, 2 et 3 du tableau 5.

TABLEAU 5

| Lot | Dose d'interleukine-2 glycosylée administrée (U/jour) | | souris survivantes au bout de 30 jours |
|---|---|---|---|
| 0 témoin | 0 | jours 10-14 <br> 0 | 0/5 |
| 1 | jours 3-7 <br> 100 | jours 10-14 <br> 5 000 | 4/5 |
| 2 | | jours 10-14 | 3/5 |
| | 0 | 5 000 | |
| 3 | jours 5-7 <br> 100 | jours 10-14 <br> 5 000 | 5/5 |

Ces résultats montrent de façon très nette l'activité antitumorale importante de l'interleukine-2 glycosylée.

## EXEMPLE 11 - ETUDE TOXICOLOGIQUE DE L'INTERLEUKINE-2 GLYCOSYLEE CHEZ LA SOURIS

a) But de l'étude

Il a été montré que le principal effet toxique de l'IL-2 recombinée (produite chez E. coli) administrée à l'homme ou à la souris, seule (M. Rosenstein et al., (1986) J. Immunol., 137, 1735-1742) ou avec des cellules LAK (S.E. Ettinghausen (1988) J. Natn Cancer Inst. 80, 3, 177-188), se manifestait par une extravasation de liquide intravasculaire et donc une rétention de ce liquide dans certains organes (thymus, rate, poumons, foie, reins).

Aussi, le but de cette étude est d'évaluer la toxicité éventuelle de l'interleukine-2 glycosylée de type rétention de liquide intravasculaire chez la souris BALB/C par la voie intrapéritonéale, après 3 administrations quotidiennes pendant 3 jours et 6 jours.

b) Protocole expérimental

Le protocole expérimental suivi correspond à celui décrit par M. Rosenstein (M. Rosenstein et al. (1986) J. Immunol, 137, 1735-1742).

α) Animaux - Conditions d'environnement

* Espèce : souris BALB/C
* Age des animaux en début d'étude : environ 4 semaines
* Acclimatation : 1 semaine
* Nombre d'animaux : 120 femelles

23

* Poids des animaux au début de l'étude : 20 g
* Méthode d'identification : marquage aux oreilles
* Alimentation : aliment complet A04C, commercialisé par la Société V.A.R., à volonté
* Abreuvement : eau de ville à volonté
* Conditions d'environnement :
  - température à 22°C (± 2°C)
  - hygrométrie à 60 % (± 10 %)
  - photopériode 12/24 h
* Habitat : cages en acier inoxydable. Les animaux sont répartis en groupes de 12 de même lot.

$\beta$) Traitement des animaux

L'IL-2 glycosylée isolée comme décrit à l'exemple 5, a été administrée parvoie intrapéritonéale en trois administrations quotidiennes (matin, midi, soir) durant 3 jours pour les lots 0, 2, 3, 4 et 5 et durant 6 jours pour les lots 1, 6, 7, 8 et 9, dans du sérum normal de souris BALB/C dilué au 80$^{ème}$ dans du tampon PBS (Phosphate Buffer Saline). Le volume administré est 0,5 ml.

Le tableau ci-après précise les doses administrées aux différents lots de 12 souris.

TABLEAU

| LOT | Dose en IL-2 glycosylée par administration | | Dose totale administrée |
| --- | --- | --- | --- |
| | $10^3$ U | $10^3$ U/kg | $10^3$ U/kg |
| 0 | 0,000 | 0,000 | 0,000 |
| 1 | 0,000 | 0,000 | 0,000 |
| 2 | 3,250 | 162,5 | 1462,5 |
| 3 | 12,500 | 625 | 5625,00 |
| 4 | 50,000 | 2,500 | 22500 |
| 5 | 200,000 | 10,000 | 90,000 |
| 6 | 3,250 | 162,5 | 2925,000 |
| 7 | 12,500 | 625 | 11,250 |
| 8 | 50,000 | 2500 | 45,000 |
| 9 | 200,00 | 10000 | 180,000 |

Les animaux des lots 0, 2, 3, 4 et 5 ont été sacrifiés au bout du 4$^{ème}$ jour, ceux des lots 1, 6, 7 et 8 le 7$^{ème}$ jour.

$\gamma$) Examens pratiques

. Examens cliniques

Les animaux ont été observés chaque jour pendant toute la durée de l'étude.
Les examens décrits ci-dessous ont été pratiqués uniquement sur les 6 premiers animaux de chaque lot.

. Examens à l'autopsie (jours 4 et 7)

Les souris anesthésiées au pentobarbital.

. Examens sanguins

Les échantillons sanguins ont été prélevés à l'autopsie pour analyse biochimique, à l'aorte abdominale.

Etude anatomique

Les organes foie, rate, reins, thymus et poumons ont été prélevés et pesés pour déceler une toxicité éventuelle de type rétention de fluide.

Détermination des poids frais : les organes ont été pesés dans des flacons à lyophiliser ; chaque flacon (un par animal et par organe) a été préalablement pesé avant d'y introduire l'organe.

Détermination des poids secs : les organes ont été congelés à -80°C puis lyophilisés pendant 4 jours dans un lyophilisateur.

. Examens particuliers

Ces examens ont été pratiqués sur les 6 derniers animaux de chaque lot. Au jour 4 pour les lots 0, 2, 3, 4 et 5 et au jour 7 pour les lots 1, 6, 7, 8 et 9, les souris ont reçu par la voie intraveineuse 2 microcuries d'albumine bovine sérique radiomarquée à l'iode 125 (activité spécifique de 5 microcuries par mg).

Deux heures après l'injection, les souris ont été sacrifiées et les organes poumons, reins, foie, thymus et rate ont été prélevés, pesés et introduits dans des tubes PVC de 5 ml. Ainsi, la radioactivité de chaque organe a pu être mesurée au compteur gamma.

L'albumine sérique bovine radio iodée peut être considérée comme un marqueur de l'extravasation de liquide intravasculaire (1). Ainsi, si un organe x d'une souris traitée à l'IL-2 est plus radioactif que celui d'une souris témoin, on peut alors être conduit à penser que l'IL-2 a une action sur la rétention de liquide intravasculaire dans l'organe considéré.

c) Résultats

. Examens cliniques

Le comportement des animaux n'a pas été modifié et aucun cas de mortalité n'a été relevé.

. Examens sanguins

L'évaluation des paramètres biochimiques (protéines totales, albumine, triglycérides, urée et créatinine) n'a pas mis en évidence de problèmes de toxicité hépatique ou rénale.

Etude comparative des poids frais et secs des organes thymus, foie, poumons, rate et reins

Les poids frais du thymus et des reins n'augmentent pas au bout de 3 ou de 6 jours de traitement, quelles que soient les doses d'IL-2.

Si l'on note une nette augmentation des poids frais de la rate, des poumons et du foie, en particulier au bout de 6 jours de traitement, le pourcentage poids sec sur poids frais ne varie pas : ceci signifie que la proportion en eau dans ces organes reste constante et traduit une hyperplasie lymphoïde inhérente à l'activité pharmacologique de l'IL-2.

Etude cinétique de l'éventuelle extravasation induite par l'IL-2 à l'aide d'albumine radiomarquée

Le taux d'albumine sérique bovine radiomarquée à l'iode 125 ne varie pas significativement dans les organes étudiés, thymus, foie, poumons, rate et reins, quelles que soient la dose et la durée du traitement et très peu dans la rate.

d) Conclusion

L'étude toxicologique réalisée sur la souris BALB/C a permis de constater l'absence d'effet toxique important de l'IL-2 glycosylée. En particulier, elle a mis en évidence à l'aide d'un protocole qui correspond à celui utilisé par M. Rosenstein l'absence d'extravasation de liquide intravasculaire, telle que décrite avec l'IL-2 coli.

EXEMPLE 12 - ETUDE TOXICOLOGIQUE DE L'INTERKEULINE-2 GLYCOSYLEE CHEZ LE RAT

a) But de l'étude

Cette étude a pour but d'évaluer la toxicité éventuelle de l'IL-2 glycosylée et en particulier la formation d'oedèmes chez le rat Sprague Dawley.

b) Méthode expérimentale

L'interleukine-2 glycosylée isolée comme décrit dans l'exemple 5 a été injectée à des lots de 6 rats (3 mâles et 3 femelles) par la voie intrapéritonéale aux doses de 2 millions, 10 millions et 50 millions d'UI/kg/j (soit 0,1, 0,5 et 2,5 mg/kg/j) pendant trois jours.

Simultanément, les animaux ont reçu du chlorure de sodium par la voie orale à la dose de 1 g/kg/j.

Le $3^{\text{ème}}$ jour, les animaux ont reçu une surcharge hydrique de 10 ml/kg par la voie orale, puis ont été placés dans une cage à métabolisme afin de mesurer la diurèse de 24 heures.

Chaque lot a été comparé à un lot témoin non traité et à un lot traité uniquement avec le chlorure de sodium.

c) Résultats

Aucune anomalie clinique n'a été observée.

Les animaux ont été pesés avant le début de l'étude, puis aux jours 3 et 4. Aucune différence entre les lots n'a été observée.

Les examens sanguins (hématocrite, urée, créatinine, protéines totales, albumine, cholestérol, triglycérides, GPT, LDH, sodium, potassium, chlorures et calcium) effectués au jour 4 ont montré une légère diminution avec effet-dose des protéines et de l'albumine plasmatiques.

L'étude de l'élimination urinaire :

.   volume
.   examen semi-quantitatif à l'aide de bandelettes réactives (pH, densité, protéines, glucose, corps cétoniques, bilirubine, sang et urobilinogène)
.   dosage des ions sodium, potassium, chlorures et de la créatinine

n'a pas montré d'anomalie.

La pesée de quelques organes (foie, rate, reins et thymus) a montré une légère augmentation du poids de la rate avec effet-dose.

L'autopsie n'a révélé aucune lésion macroscopique.

L'examen en microscopie optique a montré que l'IL-2 a induit une activation du système lymphoïde :

-   hyperplasie minime des manchons lymphoïdes périartériolaires et des zones marginales spléniques chez trois animaux traités à la dose de $50.10^6$ UI/kg/j
-   plasmocytose ganglionnaire plus fréquente chez les animaux traités, mais sans effet-dose.

d) Conclusion

Cette étude toxicologique sur les rats Sprague Dawley a mis en évidence l'absence d'effet toxique important de l'IL-2 glycosylée à une dose 5 fois plus importante (2,5 mg/kg/j contre 0,5 mg/kg/j) que la dose létale observée par Y. HARADA (Harada Y. (1987) Preclinical Safety of Biotechnology Products intended for human use - P. 127-142 - Alan R. Liss. Inc.) avec l'IL-2 coli.

L'IL-2 glycosylée est donc, tant chez la souris que chez le rat, beaucoup moins toxique que l'IL-2 coli.

EXEMPLE 13 - ESSAIS DE FORMULATION GALENIQUE DE L'INTERLEUKINE-2 GLYCOSYLEE

a) Méthodes expérimentales

Des essais de lyophilisation de la solution aqueuse de phosphate de sodium de pH 6,5 contenant l'IL-2 glycosylée, obtenue comme décrit dans l'exemple 5 à l'issue ont été effectués en présence ou en l'absence de différents excipients présents dans des proportions variables. On a ensuite reconstitué le soluté, observé son aspect, mesuré son pH et dosé la quantité d'IL-2 glycosylée présente par HPLC sur colonne de phase inverse (cf. exemple 5-3) et par mesure de l'activité CTLL-2. Ces opérations ont été effectuées immédiatement après la lyophilisation après 1 an de conservation du lyophilisat à une température de 4°C ainsi qu'après 3 mois de conservation à une température de 25°C et après 3 mois de conservation à une température de 37°C.

b) Résultats

La lyophilisation d'une solution aqueuse de phosphate de sodium contenant 80 $\mu$g/ml d'IL-2 glycosylée sans excipient permet d'obtenir, après reconstitution immédiate du soluté par addition d'eau pour préparation injectable, une solution limpide et incolore qui contient plus de 80 % de l'activité CTLL-2 initiale. Après 6 mois de conservation du lyophilisat, la solution reconstituée contient environ 50 % de l'activité CTLL-2 initiale.

L'addition lors de la lyophilisation à une solution aqueuse de phosphate de sodium contenant 80 $\mu$g/ml d'IL-2 glycosylée présentant une activité spécifique CTLL-2 voisine de 18 x 10$^6$ U/mg de 10 mg/ml de gélatine hydrolysée (ROUSSELOT Réf. DSF), de 1mg de polyéthylène glycol de poids moléculaire moyen de 6 000 (HOECHST Réf. DAB 8), ou de 10 mg/ml d'albumine sérique humaine (SIGMA Réf. 3782) permet, après 1 an de conservation du lyophilisat à 4°C de retrouver dans le soluté reconstitué limpide et de pH voisin de 6,5 par dosage chromatographique (de précision voisine de 3 %) :

- plus de 90 % pour le polyéthylène-glycol
- 100 % pour la gélatine hydrolysée et l'albumine sérique humaine

avec dans tous les cas une activité CTLL-2 spécifique voisine de 18 x 10$^6$ U/mg (d'IL-2 dosé par HPLC). Pour ces deux derniers excipients l'activité CTLL-2 par ml de soluté a donc gardé une valeur identique à sa valeur initiale.

L'addition lors de la lyophilisation à une solution aqueuse de phosphate de sodium contenant 450 $\mu$g/ml d'IL-2 glycosylée d'activité spécifique CTLL-2 voisine de 18 x 10$^6$ U/ml de 10 mg/ml de gélatine hydrolysée (ROUSSELOT Réf. DSF) et de 10 mg/ml d'alanine apyrogène (AJINOMOTO) permet d'obtenir un lyophilisat avec une teneur en eau inférieure à 3 %. Après trois mois de conservation du lyophilisat à 4°C on retrouve dans le soluté reconstitué limpide et de pH voisin de 6,5 par dosage chromatographique (de précision voisin de 3 %) 100 % de l'IL-2 glycosylée initiale avec une activité CTLL-2 spécifique voisine de 18 x 10$^6$ U/mg (d'IL-2 dosé par HPLC).

Les résultats ci-dessus montrent que l'IL-2 glycosylée présente après lyophilisation une excellente stabilité pour des formulations pharmaceutiques acceptables. Il est probable qu'on peut conserver le lyophilisat pendant plusieurs années à une température de 4°C. On constate d'autre part que dans tous les cas on obtient un soluté reconstitué limpide à un pH physiologique sans ajout d'agent toxique ni modification chimique de la molécule pour la rendre soluble. Ces propriétés de l'IL-2 glycosylée dérivée d'E. COLI. Celle-ci perd en effet plus de 50 % de activité et donne des solutions opalescentes après lyophilisation (voir la demande de brevet européenne N° 0 158 487, p. 10). Il est donc nécessaire pour la rendre soluble d'ajouter des agents solubilisants toxiques tels que le SDS (Sodium Dodecyl Sulfate).

L'homme de l'art appréciera, à la lecture de la description ci-dessus, l'intérêt de l'IL-2 glycosylée comme médicament, et les nombreux avantages que présente cette dernière par rapport à l'IL-2 non glycosylée, par exemple dérivée d'E. coli : possibilité d'utiliser des doses plus faibles pour la même activité et meilleure tolérance à des doses fortes grâce à une plus faible toxicité, moindre immunogénicité, solubilité dans un solvant aqueux à un pH physiologique sans addition d'agent solubilisant toxique ni modification chimique de la molécule, excellente stabilité après lyophilisation dans des formulations pharmaceutiquement acceptables.

**Revendications**

1. Médicament, caractérisé en ce qu'il contient comme principe actif une interleukine-2 glycosylée purifiée constituée d'un mélange d'interleukine-2 glycosylée disialilée et d'interleukine-2 glycosylée monosialilée, seule ou en association avec d'autres principes actifs, dans un excipient pharmaceutiquement acceptable.

2. Médicament selon la revendication 1, caractérisé en ce que l'interleukine-2 glycosylée disialilée est majoritaire dans l'interleukine-2 purifiée.

3. Médicament selon l'une des revendications 1 ou 2, caractérisé en ce que la pureté de l'interleukine-2 glycosylée purifiée déterminée par électrophorèse sur gel de polyacrylamide en présence de SDS, est supérieure à 99 %.

4. Médicament selon l'une des revendications 1 et 2, caractérisé en ce que la pureté de l'interleukine-2 glycosylée purifiée déterminée par électrophorèse sur gel de polyacrylamide en présence de SDS, est supérieure à 99,5 %.

5. Médicament selon l'une quelconque des revendications 1 à 4, caractérisé en ce que l'activité spécifique CTLL-2 de l'interleukine-2 glycosylée purifiée est supérieure à 15 X 10$^6$ U/mg.

6. Médicament selon l'une quelconque des revendications 1 à 5, caractérisé en ce que l'interleukine-2 glycosylée purifiée est isolée à partir d'un surnageant de cellules CHO recombinées, à l'aide d'un procédé comprenant les étapes suivantes :
   a) séparation d'une fraction riche en interleukine-2 du surnageant de culture
   b) chromatographie d'échange cationique, les fractions $N_1$ et $N_2$ obtenues sont regroupées et soumises aux étapes :
   c) chromatographie d'interaction hydrophobe
   d) chromatographie d'exclusion

7. Médicament selon la revendication 6, caractérisé en ce que les cellules CHO recombinées sont des cellules dérivées de la souche DXB11 par transformation à l'aide d'un vecteur d'expression pour un précurseur de l'interleukine-2 la DHFR.

8. Médicament selon l'une des revendications 6 ou 7, caractérisé en ce que les cellules CHO recombinées sont cultivées dans un milieu synthétique pauvre en protéines.

9. Médicament selon la revendication 8, caractérisé en ce que le milieu synthétique pauvre en protéines contient de la polyvinylpyrrolidone.

10. Médicament selon l'une quelconque des revendications 6 à 9, caractérisé en ce que la séparation de l'étape a) est effectuée par double filtration entre une membrane (1) ayant un seuil d'arrêt supérieur à 30 kDa, de préférence comprise entre 50 et 150 kDa, et une membrane (2) ayant un seuil d'arrêt inférieur à 10 kDa de préférence entre 5 et 10 kDa.

11. Médicament selon l'une quelconque des revendications 6 à 10, caractérisé en ce que l'on effectue l'étape d'échange cationique dans un intervalle de pH compris entre 4,5 et 6,5, de préférence entre 5,2 et 5,7.

12. Médicament selon l'une quelconque des revendications 6 à 11, caractérisé en ce que l'étape d'échange cationique est précédée d'une étape de chromatographie d'échange anionique.

13. Médicament selon la revendication 12, caractérisé en ce que l'on effectue l'étape de chromatographie d'échange anionique dans un intervalle de pH compris entre 5,5 et 8,5 de préférence entre 6,5 et 8,2.

14. Médicament selon l'une des revendications 6 à 13, caractérisé en ce qu'on effectue l'étape d'interaction hydrophobe dans un intervalle de pH compris entre 4,5 et 8,0, de préférence entre 6,0 et 8,0.

15. Médicament selon l'une quelconque des revendications 6 à 14, caractérisé en ce que l'on effectue l'étape d'interaction hydrophobe sur un support chromatographique permettant la désorption de l'interleukine-2 sans addition d'agents chaotropiques, de solvants organiques ou de solvants aqueux ayant un pH inférieur à 4,5 ou supérieur à 8,0.

16. Médicament selon l'une quelconque des revendications 6 à 15, caractérisé en ce que l'on effectue l'étape de chromatographie d'exclusion sur un support ayant une gamme de fractionnement comprise entre 1 et 250 kDa.

17. Médicament selon l'une quelconque des revendications 1 à 16, caractérisé en ce que l'interleukine-2 glycosylée permet d'obtenir une solution reconstituée limpide à un pH physiologique, après lyophilisation d'une solution aqueuse la contenant de pH 6,5 et reconstitution immédiate du soluté.

18. Médicament selon l'une quelconque des revendications 1 à 17, caractérisé en ce que l'interleukine-2 glycosylée conserve son activité CTLL-2 initiale après lyophilisation dans une solution aqueuse de pH 6,5, additionnée de gélatine hydrolysée ou d'albumine sérique humaine, conservation de lyophilisat à une température de 4° C pendant un an et reconstitution du soluté.

19. Médicament selon l'une quelconque des revendications 1 à 18, caractérisé en ce que l'IL-2 glycosylée conserve son activité CTLL-2 initiale après lyophilisation dans une solution aqueuse de pH 6,5, additionnée de gélatine hydrolysée et d'alanine, conservation de lyophilisat à une température de 25° C pendant 3 mois et reconstitution du soluté.

20. Médicament selon l'une quelconque des revendications 1 à 19, caractérisé en ce que l'IL-2 glycosylée conserve son activité CTLL-2 après lyophilisation dans une solution aqueuse de pH 6,5, additionnée de gélatine hydrolysée et d'alanine, conservation du lyophilisat à une température de 37° C pendant 3 mois et reconstitution du soluté.

21. Médicament selon l'une quelconque des revendications 1 à 20, caractérisé en ce que l'excipient pharmaceutiquement acceptable contient de la gélatine hydrolysée et de l'alanine.

**Claims**

1. Drug, characterized in that it contains, as the active principle, a purified glycosylated interleukin-2 constituted of a mixture of disialilated glycosylated interleukin-2 and monosialilated glycosylated interleukin-2, alone or in association with other active principles, in a pharmaceutically acceptable excipient.

2. Drug according to claim 1, characterized in that the disialilated glycosylated interleukin-2 is in the highest proportion in the purified interleukin-2.

3. Drug according to one of claims 1 or 2, characterized in that the purity of the purified glycosylated interleukin-2 determined by electrophoresis on polyacrylamide gel in the presence of SDS, is above than 99%.

4. Drug according to one of claims 1 and 2, characterized in that the purity of the purified glycosylated interleukin-2 determined by electrophoresis on polyacrylamide gel in the presence of SDS, is above than 99.5%.

5. Drug according to any one of claims 1 to 4, characterized in that the glycosylated interleukin-2 has a specific CTLL-2 activity above $15 \times 10^6$ U/mg.

6. Drug according to any one of claims 1 to 5, characterized in that the purified glycosylated interleukin-2 is isolated from a supernatant of recombined CHO cells, by a process comprising the following steps:
   a) separation of a fraction rich in interleukin-2 from the culture supernatant
   b) cation exchange chromatography, the obtained fractions $N_1$ and $N_2$ are regrouped and submitted to the steps:
   c) hydrophobic interaction chromatography
   d) exclusion chromatography

7. Drug according to claim 6, characterized in that the recombined CHO cells are cells derived from the strain DXB11 by transformation by an expression vector for a precursor of interleukin-2 and DHFR.

8. Drug according to one of claims 6 or 7, characterized in that the recombined CHO cells are cultivated in a synthetic medium low in proteins.

9. Drug according to claim 8, characterized in that the synthetic medium low in proteins contains polyvinyl pyrrolidone.

10. Drug according to any one of claims 6 to 9, characterized in that the separation in step a) is brought about by double filtration between a membrane (1) having a stop threshold above 30 kDa, preferably between 50 and 150 kDa, and a membrane (2) having a stop threshold below 10 kDa, preferably between 5 and 10 kDa.

11. Drug according to any one of claims 6 to 10, characterized in that the cation exchange step is brought about at a pH in the range between 4.5 and 6.5, preferably between 5.2 and 5.7.

12. Drug according to any one of claims 6 to 11, characterized in that the cation exchange step is preceded by an anion exchange chromatography step.

13. Drug according to claim 12, characterized in that the anion exchange chromatography step is brought about at a pH in the range between 5.5 and 8.5, preferably between 6.5 and 8.2.

14. Drug according to one of claims 6 to 13, characterized in that the hydrophobic interaction step is carried out at a pH in the range between 4.5 and 8.0, preferably between 6.0 and 8.0.

15. Drug according to any one of claims 6 to 14, characterized in that the hydrophobic interaction step is brought about on a chromatographic support for desorption of interleukin-2 without addition of chaotropic agents or organic solvents or aqueous solvents at a pH below 4.5 or above 8.0.

16. Drug according to any one of claims 6 to 15, characterized in that the exclusion chromatography step is carried out on a support having a fractionation range between 1 and 250 kDa.

17. Drug according to any one of claims 1 to 16, characterized in that the glycosylated interleukin-2 can be used to obtain a clear reconstituted solution at a physiological pH after lyophilisation of an aqueous solution containing it at pH 6.5 and immediate reconstitution of the solute.

18. Drug according to any one of claims 1 to 17, characterized in that the glycosylated interleukin-2 retains its initial CTLL-2 activity after lyophilisation in an aqueous solution of pH 6.5 to which hydrolysed gelatin or human serum albumin has been added, preservation of the lyophilised product at a temperature of 4°C for one year, and reconstitution of the solute.

19. Drug according to any one of claims 1 to 18, characterized in that the glycosylated interleukin-2 retains its initial CTLL-2 activity after lyophilisation in an aqueous solution of pH 6.5 to which hydrolysed gelatin and alanine have been added, preservation of the lyophilised product at a temperature of 25°C for 3 months, and reconstitution of the solute.

20. Drug according to any one of claims 1 to 19, characterized in that the glycosylated interleukin-2 retains its CTLL-2 activity after lyophilisation in an aqueous solution of pH 6.5 to which hydrolysed gelatin and alanine have been added, preservation of the lyophilised product at a temperature of 37°C for 3 months, and reconstitution of the solute.

21. Drug according to any one of claims 1 to 20, characterized in that the pharmaceutically acceptable excipient contains hydrolysed gelatin and alanine.

## Patentansprüche

1. Medikament, dadurch gekennzeichnet, daß es als Wirkstoff ein gereinigtes glycosyliertes Interleukin 2, das durch eine Mischung von disialisiertem glycosyliertem Interleukin 2 und monosialisiertem glycosyliertem Interleukin 2 gebildet ist, entweder allein oder in Kombination mit anderen Wirkstoffen in einem pharmazeutisch akzeptablen Exzipienten enthält.

2. Medikament nach Anspruch 1, dadurch gekennzeichnet, daß disialisiertes glycosyliertes Interleukin 2 die Mehrheit im gereinigten Interleukin 2 bildet.

3. Medikament nach einem der Ansprüche 1 oder 2, dadurch gekennzeichnet, daß die Reinheit des gereinigten glycosylierten Interleukin 2, bestimmt durch Elektrophorese auf Polyacrylamidgel in Anwesenheit von SDS, über 99 % beträgt.

4. Medikament nach einem der Ansprüche 1 und 2, dadurch gekennzeichnet, daß die Reinheit des gereinigten glycosylierten Interleukin 2, bestimmt durch Elektrophorese auf Polyacrylamidgel in Anwesenheit von SDS, über 99,5 % beträgt.

5. Medikament nach einem der Ansprüche 1 bis 4, dadurch gekennzeichnet, daß die spezifische CTLL 2-Aktivität des gereinigten glycosylierten Interleukin 2 über $15 \times 10^6$ E/mg beträgt.

6. Medikament nach einem der Ansprüche 1 bis 5, dadurch gekennzeichnet, daß das gereinigte glycosylierte Interleukin 2 aus einem Überstand von rekombinanten CHO-Zellen mittels eines Verfahrens isoliert wird, das die folgenden Schritte umfaßt:

a) die Abtrennung einer Interleukin 2-reichen Fraktion vom Kulturüberstand

b) eine Kationenaustauschchromatografie, die erhaltenen Fraktionen $N_1$ und $N_2$ werden gepoolt und den folgenden Schritten unterzogen:

c) einer hydrophoben Wechselwirkungschromatografie

d) einer Exklusionschromatografie.

7. Medikament nach Anspruch 6, dadurch gekennzeichnet, daß die rekombinanten CHO-Zellen Zellen sind, die vom Stamm DXB11 durch Transformation mit Hilfe eines Expressionsvektors für einen Präkursor von Interleukin 2 und von DHFR abgeleitet sind.

8. Medikament nach einem der Ansprüche 6 oder 7, dadurch gekennzeichnet, daß die rekombinanten CHO-Zellen in einem synthetischen Medium, das arm an Proteinen ist, kultiviert werden.

9. Medikament nach Anspruch 8, dadurch gekennzeichnet, daß das proteinarme synthetische Medium Polyvinylpyrrolidon enthält.

10. Medikament nach einem der Ansprüche 6 bis 9, dadurch gekennzeichnet, daß die Abtrennung aus Schritt a) durch doppelte Filtration zwischen einer Membran (1) mit einer Aufhaltschwelle von über 30 kDa, vorzugsweise zwischen 50 und 150 kDa, und einer Membran (2) mit einer Aufhaltschwelle von unter 10 kDa, vorzugsweise zwischen 5 und 10 kDa, erfolgt.

11. Medikament nach einem der Ansprüche 6 bis 10, dadurch gekennzeichnet, daß man den Kationenaustauschschritt in einem pH-Bereich zwischen 4,5 und 6,5, vorzugsweise zwischen 5,2 und 5,7, ausführt.

12. Medikament nach einem der Ansprüche 6 bis 11, dadurch gekennzeichnet, daß vor dem Kationenaustauschschritt ein Anionenaustauschchromatografieschritt ausgeführt wird.

13. Medikament nach Anspruch 12, dadurch gekennzeichnet, daß man den Anionenaustauschchromatografieschritt in einem pH-Bereich zwischen 5,5 und 8,5, vorzugsweise zwischen 6,5 und 8,2, ausführt.

14. Medikament nach einem der Ansprüche 6 bis 13, dadurch gekennzeichnet, daß man den hydrophoben Wechselwirkungsschritt in einem pH-Bereich zwischen 4,5 und 8,0, vorzugsweise zwischen 6,0 und 8,0, ausführt.

15. Medikament nach einem der Ansprüche 6 bis 14, dadurch gekennzeichnet, daß man den hydrophoben Wechselwirkungsschritt auf einem chromatografischen Träger ausführt, der die Desorption von Interleukin 2 ohne Zugabe von chaotropischen Mitteln, organischen Lösungsmitteln oder wässerigen Lösungsmitteln mit einem pH-Wert von unter 4,5 oder über 8,0 gestattet.

16. Medikament nach einem der Ansprüche 6 bis 15, dadurch gekennzeichnet, daß man den Exklusionschromatografieschritt auf einem Träger mit einem Fraktionierbereich zwischen 1 und 250 kDa ausführt.

17. Medikament nach einem der Ansprüche 1 bis 16, dadurch gekennzeichnet, daß das glycosylierte Interleukin 2 den Erhalt einer klaren rekonstituierten Lösung mit einem physiologischen pH-Wert nach der Lyophilisierung einer dieses enthaltenden wässerigen Lösung mit pH 6,5 und sofortigen Rekonstitution des gelösten Stoffs gestattet.

18. Medikament nach einem der Ansprüche 1 bis 17, dadurch gekennzeichnet, daß das glycosylierte Interleukin 2 nach Lyophilisierung in einer wässerigen Lösung mit pH 6,5, die mit hydrolysierter Gelatine oder Humanserumalbumin versetzt ist, einjähriger Konservierung des Lyophilisats bei einer Temperatur von 4°C und Rekonstituierung des gelösten Stoffs seine ursprüngliche CTLL-2-Aktivität beibehält.

19. Medikament nach einem der Ansprüche 1 bis 18, dadurch gekennzeichnet, daß das glycosylierte IL 2 nach Lyophilisierung in einer wässerigen Lösung mit pH 6,5, die mit hydrolysierter Gelatine und Alanin versetzt ist, dreimonatiger Konservierung des Lyophilisats bei einer Temperatur von 25°C und Rekonstituierung des gelösten Stoffs seine ursprüngliche CTLL-2-Aktivität beibehält.

**20.** Medikament nach einem der Ansprüche 1 bis 19, dadurch gekennzeichnet, daß das glycosylierte IL 2 nach Lyophilisierung in einer wässerigen Lösung mit pH 6,5, die mit hydrolysierter Gelatine und Alanin versetzt ist, dreimonatiger Konservierung des Lyophilisats bei einer Temperatur von 37°C und Rekonstituierung des gelösten Stoffs seine CTLL 2-Aktivität beibehält.

**21.** Medikament nach einem der Ansprüche 1 bis 20, dadurch gekennzeichnet, daß der pharmazeutisch akzeptable Exzipient hydrolysierte Gelatine und Alanin enthält.

FIG.1

pSV 720

EcoRI

BglⅡ

HindⅢ

(PvuⅡ)
(EcoRⅤ)

BamHI
(HpaI)

(BàlI)

BamHI

AGCTTCCACCATGGCTAGG

HindⅢ

PvuⅡ

FIG. 2

# FIG.3

ÉLECTROPHORÈSE SUR GEL DE POLYACRYLAMIDE
EN PRÉSENCE DE SDS APRÈS RÉDUCTION DES
ÉCHANTILLONS.  COLORATION À L'ARGENT

1  MÉLANGE DES 4 FORMES D'IL2
2  PRODUIT OBTENU À L'ISSUE DE L'ÉTAPE 2d)DE L'EXEMPLE 1
3  IL2N2
4  IL2N1
5  MARQUEUR DE POIDS MOLÉCULAIRE
6  IDEM 1
7  IDEM 5

# FIG.4

ANALYSE PAR HPLC SUR COLONNE DE PHASE
INVERSE DU PRODUIT OBTENU A L'ISSUE DE L'ÉTAPE 2)d)
DE L'EXEMPLE 5

# FIG. 5

ANALYSE PAR HPLC SUR COLONNE ÉCHANGEUSE DE CATIONS
DU PRODUIT OBTENU À L'ISSUE DE L'ÉTAPE 2) d) DE L'EXEMPLE 5

6,49 IL2N2

7,54 IL2N1

# FIG. 6

VALEUR PONDÉRALE MOYENNE DE LA RATE
RAPPORTÉE AU POIDS CORPOREL

VALEUR PONDÉRALE DE
LA RATE RAPPORTÉE
AU POIDS CORPOREL
$( g/100 )$

DOSE D'IL-2
GLYCOSYLÉE
ADMINISTRÉE
$( 10^6 U/kg )$

0,8

0,7

0,6

0,5

0    0,075   0,375   1,88   9,38   46,9

# FIG. 7

NOMBRE TOTAL MOYEN DE SPLÉNOCYTES
PAR ANIMAL

FIG.8

DOSAGE DES IgM
ANTI-OVALBUMINE

FIG.9

——————————————— TÉMOIN

— — — — — — — — LOT 1 : 100 U/J LES JOURS 3-7 ET 5000 UI/J LES JOURS 10-14

— • — • — • — • — LOT 2 : 5000 U/J LES JOURS 10-14

— × — × — × — × — LOT 3 : 100 U/J LES JOURS 5-7 ET 5000 UI/J LES JOURS 10-14

NOMBRE DE SOURIS

TEMPS (JOURS)